# EUROPEAN PATENT SPECIFICATION

(11) **EP 3 697 326 B1**
(45) Date of publication and mention of the grant of the patent: **01.07.2026**
(21) Application number: 18868614.1
(22) Date of filing: 16.10.2018
(51) Int. Cl.: A61B 17/22, A61B 17/00, A61B 17/08, A61F 2/01, A61F 2/82

(54) **DEVICES FOR TREATING BLOCKED BLOOD VESSELS**
VORRICHTUNGEN ZUR BEHANDLUNG VERSTOPFTER BLUTGEFÄSSE
DISPOSITIFS DE TRAITEMENT DE VAISSEAUX SANGUINS OBSTRUÉS

(30) Priority: 16.10.2017 US 201762573097 P
(43) Date of publication of application: 26.08.2020
(73) Proprietor: Shanghai Wallaby Medical Technologies Co., Inc., Pudong New Area, Shanghai 201318 (CN)
(72) Inventor: CHOE, Jerome, Los Angeles California 90012 (US); BARDSLEY, Earl, San Clemente California 93623 (US); IAN, Cheng, Merrick New York 11566 (US); FENG, Chengcheng, Irvine California 92620 (US)
(74) Representative: FRKelly
(86) International application number: PCT/US2018/056079
(87) International publication number: WO 2019/079296

(56) References cited:
- CN-A- 106 108 980
- US-A1- 2011 160 763
- US-A1- 2014 081 315
- US-A1- 2016 120 569
- US-A1- 2016 192 956
- US-B2- 9 271 820
- US-B2- 9 642 639

## Description

### CROSS-REFERENCE TO RELATED APPLICATION

This application claims priority to and the benefit of U.S. Provisional Patent Application Serial Number 62/573,097, entitled "Devices And Methods For Treating Blocked Blood Vessels," filed October 16, 2017.

### FIELD

The present teachings relate to minimally invasive catheter and methods of minimally invasive catheter delivered embolic capture devices for use in the vasculature, especially those suited for usage in the brain and vessel systems perfusing the same.

### BACKGROUND

Mechanical thrombectomy devices seek to salvage ischemic, but not yet fully infarcted, brain by restoring perfusion through the initially occluded artery. Each class of mechanical thrombectomy devices achieves recanalization through somewhat different biomechanical mechanisms.

There are three types of catheter thrombectomy devices on the market - aspiration catheters to vacuum the clot out of the vessel, lytic deliver catheters to infuse the clot with targeted thromobytic drug treatment, and mechanical thrombectomy systems that Engage and retract clot. The systems on the market may combine one or more of these attributes. There are pros and cons to each of these approaches.

The aspiration catheters employ vacuum aspiration to remove occlusive clot in acute ischemic stroke. While manual aspiration of target thrombi can be performed through any microcatheter, such as by applying suction through a bore small enough to fit within intracranial arteries. The aspiration catheters are often used in rapid single-session flow restoration for removing small, fresh, soft thrombus. However, larger and more organized thrombus can overwhelm and emboli the small aperture of a manual aspiration catheter. In addition, manual aspiration is more likely than mechanical methods to leave residual thrombus. Additionally, aspiration catheters, generally having a large profile, have difficulty in crossing lesions.

The lytic delivery catheters directed thrombolysis (CDT) is the localized delivery of lytic via a catheter to dissolve thrombus and to restore vascular flow. Although the lytic therapy offers improved outcomes versus the standard anticoagulation therapy, the lytic therapy alone is often not fast enough to resolve a critical coronary blockage as found in STEMI or in restoring flow in peripheral vasculature. In peripheral cases, a lytic-only treatment may require extended stays in the ICU and frequent angiographic re-visualizations to check the progress. To treat a large thrombus burden of the neuro vasculature, a significant systemic dose of lytic is often used before the blockage can be fully resolved. Such higher doses of lytic delivery could increase the risk of bleeding.

Mechanical thrombectomy, in conjunction with systemic thrombolysis, is currently the standard of care for the treatment of acute ischemic stroke. There are two kinds of mechanical thrombectomy systems, coil retriever and stent retriever. The coil retrievers are composed of Nitinol shape-memory wire and delivered through a microcatheter across the target clot. As the device is extruded from delivery catheter, it immediately reassumes its native coil form. The neurointerventionalist deploys the loops of the coil through the clot to engage the thrombus, and then pulls both coil and clot back into the catheter, like pulling a cork from a wine bottle. The stent retrievers are self-expanding stents that are deployed in the occluded vessel within the thrombus, engaging it and entangling it within the stent struts. The stent and thrombus are then withdrawn back into the delivery catheter.

With the capability of restoring flow in a single session, stent retriever removes a higher percentage of clot than the manual aspiration methods and can restore flow in a significantly less time than the lytic treatment alone. Despite their superiority in improving clinical outcomes in patients with acute ischemic strokes, however, stent retrievers are not without complications. Recent study has found that these devices could cause vascular damage that extends into the medial layer. Another common disadvantage of stent retriever is that stent retrieval necessarily induces clot fragmentation, which may result in distal embolization and occlusion of previously uninvolved territory. Thus, rooms for improvement US2016/192956 A1, US2014/081315 A1 and US2016/120569 A1 disclose devices of the prior art.

### SUMMARY

Claim 1 defines the invention and dependent claims disclose embodiments. No surgical methods are claimed per se. One aspect of the present teachings provides an embolic capture device. In various embodiments, the embolic capture device comprises an elongated stent body with a plurality of cells along its elongated body. The elongated stent body has a longitudinal axis extending from a proximal end to a distal end. A plurality of proximal struts joining the proximal end of stent body forming a proximal hub. Each of the plurality of cells has a proximal end and a distal end of, together forming a cell axis. And at least one cell axis forms an angle with the longitudinal axis of the elongated stent body.

Another aspect of the present teachings provides an embolic capture device. In various embodiment, the embolic capture device comprises an elongated stent body with a plurality of cells along its elongated body. The elongated stent body has a longitudinal axis extending from a proximal end to a distal end. The elongated stent body has a radially collapsed delivery profile and a radially expanded deployed profile. The elongated stent body is configure to rotate around its longitudinal axis as it transitions from its radially collapsed delivery profile to its radially expanded deployed profile.

One aspect of the present teachings provides an embolic capture device. In various embodiments, the device has an elongated stent body with a plurality of proximal struts joining its proximal end forming a proximal hub. In some embodiments, the device also has a distal net, positioned distal to the stent body and having an adjustable distance to a distal end of the stent. Another aspect of the present teachings provides a pusher shaft and a pull wire. The pusher shaft joins the proximal end of the stent bod. The pull wire extends through the proximal hub and the elongated stent body, and joining the distal net. In some embodiments, the stent body and the distal net move independently of each other so that the distance between the distal end of the stent and the distal net changes.

Another aspect of the present teachings provides an embolic capture device. In various embodiments, the device has an elongated stent body with a plurality of proximal struts joining its proximal end forming a proximal hub. In some embodiments, the device also has a distal net, positioned distal to the stent body and having an adjustable distance to a distal end of the stent. Another aspect of the present teachings provides that the device has a first configuration wherein a first distance in between the distal net and the distal end of the stent body, and a second configuration wherein a second distance in between the distal net and the distal end of the stent body.

Another aspect of the present teachings provides an embolic capture device. In various embodiments, the device has an elongated stent body with a plurality of proximal struts joining its proximal end forming a proximal hub. In some embodiments, the device also has at least one mesh net positioned within the elongated stent body. A proximal edge of the mesh net joins an inner luminal wall of the stent. A distal peak of the mesh net is approximate to the longitudinal axis of the stent body. Another aspect of the present teachings provides that the device has a first configuration where both the stent body and mesh net inside are collapsed radially, and a second configuration where both the stent body and mesh net expands radially.

Another aspect of the present teachings provides an embolic capture device. In various embodiments, the device has an elongated stent body with a plurality of proximal struts joining its proximal end forming a proximal hub. In some embodiments, the device also has at least two stabilizing struts. The proximal ends of the stabilizing struts attach to an inner luminal surface of the stent body. The distal ends of the stabilizing struts join together forming a hub. Another aspect of the present teachings provides a pusher shaft and a pull wire. The pusher shaft joins the proximal end of the stent body. The pull wire extends through the proximal hub, the elongated stent body, and joins the hub of the stabilizing struts.

Another aspect of the present teachings provides an embolic capture device. In various embodiments, the device has an elongated stent body with a plurality of proximal struts joining its proximal end forming a proximal hub. In some embodiments, the device also has at least two directional struts. The proximal ends of the directional struts join together forming a first hub. The distal ends of the directional struts join together forming a second hub. A middle point of each strut attaches to an inner luminal surface of the stent body. The middle point is distal and radially outward from the proximal end. Another aspect of the present teachings provides a pusher shaft and a pull wire. The pusher shaft joins the proximal end of the stent body. The pull wire extends through the proximal hub of the stent body, the elongated stent body, and joins the first hub of the directional struts.

Another aspect of the present teachings provides an embolic capture device. In various embodiments, the device has a generally elongated body. The generally elongated body has at least one continuous portion and at least one discontinuous portion. In some embodiments, the continuous portion has open cell surface structure. The discontinuous portion is configured to be larger than one open cell. Another aspect of the present teachings provides that the discontinuous portion spaces along the generally elongated body of the device in a linear fashion.

Another aspect of the present teachings provides an embolic capture device. In various embodiments, the device has an elongated wire having a distal portion. In some embodiments, a ribbon wrapped around the distal portion of the elongated wire in a helical fashion. Another aspect of the present teachings provides that the device has a first configuration where the ribbon is tightly wrapped around the distal portion of the wire, and a second configuration where the ribbon unwraps and expands radially.

### BRIEF DESCRIPTION OF THE DRAWINGS

FIG. 1 is a perspective view of an exemplary medical device in accordance with the present teachings.
FIG. 2 is a perspective view of an exemplary medical device in accordance with the present teachings.
FIGs. 3A-3C are perspective views of various cell structure design along the elongated body of the exemplary medical device of FIGs. 1 and 2 in accordance with the present teachings.
FIG. 3D is a perspective view of an exemplary medical device in accordance with the present teachings.
FIGs. 3E-3F are perspective views of various cell structure design along the elongated body of the exemplary medical device of FIG. 3D in accordance with the present teachings.
FIG. 3G is a perspective view of an exemplary medical device in accordance with the present teachings.
FIGs. 3H-3L are perspective views of various cell structure design along the elongated body of the exemplary medical device of FIGs. 1 and 2 in accordance with the present teachings.
FIG. 4 is a perspective view of an exemplary medical device in accordance with the present teachings.
FIGs. 5A-5C are perspective views of an exemplary medical device in accordance with the present teachings.
FIG. 6 is a perspective view of an exemplary medical device in accordance with the present teachings.
FIGs. 7-8 are perspective views of an exemplary medical device in accordance with the present teachings.
FIGs. 9A-9B are perspective views of an exemplary medical device in accordance with the present teachings.
FIG. 10 is a perspective view of an exemplary medical device in accordance with the present teachings.
FIG. 11 is a perspective view of an exemplary medical device in accordance with the present teachings.
FIGs. 12A-12B are perspective views of an exemplary medical device in accordance with the present teachings.
FIGs. 13A-13B are perspective views of an exemplary medical device in accordance with the present teachings.
FIG. 14 is a perspective view of an exemplary medical device in accordance with the present teachings.
FIG. 15 is a perspective view of an exemplary medical device in accordance with the present teachings.
FIG. 16 is a perspective view of an exemplary medical device in accordance with the present teachings.
FIGs. 17A-17B are perspective views of an exemplary medical device in accordance with the present teachings.
FIG. 18 is a perspective view of an exemplary medical device in accordance with the present teachings.
FIG. 19 is a perspective view of an exemplary medical device in accordance with the present teachings.
FIG. 20 is a perspective view of an exemplary medical device in accordance with the present teachings.
FIGs. 21A-21B are perspective views of an exemplary medical device in accordance with the present teachings.
FIGs. 22A-22B are perspective views of an exemplary medical device in accordance with the present teachings.
FIG. 23 is a perspective view of an exemplary medical device in accordance with the present teachings.
FIG. 24 is a perspective view of an exemplary medical device in accordance with the present teachings.
FIG. 25 is a perspective view of an exemplary medical device in accordance with the present teachings.
FIGs. 26A-26B are perspective views of an exemplary medical device in accordance with the present teachings.
FIG. 27 is a perspective view of an exemplary medical device in accordance with the present teachings.
FIGs. 28A-28C are perspective views of an exemplary medical device in accordance with the present teachings.

### DETAILED DESCRIPTION

In one aspect, the present teachings are described more fully hereinafter with reference to the accompanying drawings, which show certain embodiments of the present teachings. The present teachings may, however, be embodied in many different forms and should not be construed as limited to the embodiments set forth herein; rather, these embodiments are provided to illustrate various aspects of the present teachings. Like numbers refer to like elements throughout.

In one aspect, the present teachings provide catheter-based emboli removal systems. In some embodiments, a system of the present teachings is used to remove blood clots from vessels in the body. In some embodiments, the vessels are veins. In some embodiments, the system is used to treat, in veins, deep vein thrombosis (DVT), and in arteries, pulmonary embolism (PE), ST-elevated myocardial infarction (STEMI) and ischemic stroke. In some embodiments, the systems can also quickly clear dialysis arteriovenous grafts, which are prone to thrombus formation. According to some embodiments, when the catheter-based emboli removal system of the present teachings is deployed into a blood vessel, the emboli removal device is expanded and moved proximally along the vessel so that the embolus is substantially contained with a mesh basket of the emboli removal device. Specifically, the present teachings provide a device/system and methods of removing neurocranial emboli without causing any distal complication arising from the dislodgement of larger pieces of a recovered embolus distal to the location of the original embolus.

As used herein, the terms "radially outward" and "radially away" means any direction which is not parallel with the central axis. For example, considering a cylinder, a radial outward member could be a piece of wire or a loop of wire that is attached or otherwise operatively coupled to the cylinder that is oriented at an angle greater than 0° relative to the central longitudinal axis of the cylinder.

As used herein, the term "lumen" means a canal, duct, generally tubular space or cavity in the body of a subject, including veins, arteries, blood vessels, capillaries, intestines, and the like. The term "lumen" can also refer to a tubular space in a catheter, a microcatheter, or the like in a device.

As used herein the term "proximal" shall mean closest to the operator (less into the body) and "distal" shall mean furthest from the operator (further into the body). In positioning the medical device from a downstream access point, distal is more upstream and proximal is more downstream.

As used herein the term "emboli" used herein can be clot, thrombus or the like, and these terms may be used interchangeably.

As explained in further detail below, various embodiments of the present teachings provide medical devices/system for removing blood clots from a vessel in the body. In some embodiments, the medical devices/system may include an embolic capture device or means configured to capture the clot. In some embodiments, a pusher shaft joins the embolic capture device. In some embodiments, the pusher shaft pushes and/or pulls the embolic capture device. In some embodiments, the embolic capture device according to the present teachings may be extended into an elongated profile for percutaneous delivery, and resume to a radially expanded deployment profile for capturing the clot, and be extended into a second elongated profile to retrieve the clot. As used in this application, unless otherwise indicated, the term "vessel" refers to a blood vessel, including an artery, an arteriole, a capillary, a venule, a vein or a network of any of the combinations of the foregoing.

In another aspect, the present teachings disclose an embolic capture device for intracranial use. According to some embodiments, the embolic capture device has a general profile of a stent that is flexible and atraumatic, and is available in various lengths and diameters, thin-walled, and/or radiopaque. In some embodiments, the stent is configured to be precisely delivered, retrieved, and repositioned. In some embodiments, the stent is flexible enough to be delivered via a microcatheter and to be placed in a small vessel but has sufficient radial forces to conform to the vessel wall geometry when deployed.

In another aspect, the present teachings disclose an embolic capture device with an elongated delivery profile. In some embodiments, the embolic capture device has an expanded deployed profile. As described in detail below, in some embodiments, the embolic capture device can have a straightened, elongated, low-profile delivery configuration suitable for delivery via a delivery system. In some embodiments, the deployed configuration of the embolic capture device substantially engages the blood vessel within which it is deployed. When an embolic capture of the present teachings is used to retrieve an emboli, a positioning wire is first threaded through the blood vessel across a blood clot. A microcatheter then threads over the positioning wire and having its distal end positioned distal to the clot. The positioning wire is then removed, followed by a pusher shaft joining to a proximal end of an elongated embolic capture device extending through the lumen of microcatheter. While holding elongated embolic capture device steady, a clinician withdraws the microcatheter proximally to uncover the device. Once outside of the microcatheter, the embolic capture device expands to stretch the wall of the artery so blood can flow. In one embodiment, the device is deployed distal to the clot. In another embodiment, the device is deployed across the clot. In some embodiments, to retrieve the clot, the clinician pulls the pusher shaft proximally, the embolic capture device is pulled proximally back, carrying the blood clot back into a larger catheter, guide catheter, or distal access catheter (DAC).

The techniques disclosed for delivering and deploying the embodiments described herein are only examples. It should be understood that other techniques can be used instead of, or in combination with, these teachings. For example, the techniques used to deploy an embodiment of the devices described herein depend on the particular features of the device, the delivery system, and the anatomy in which the device is being deployed.

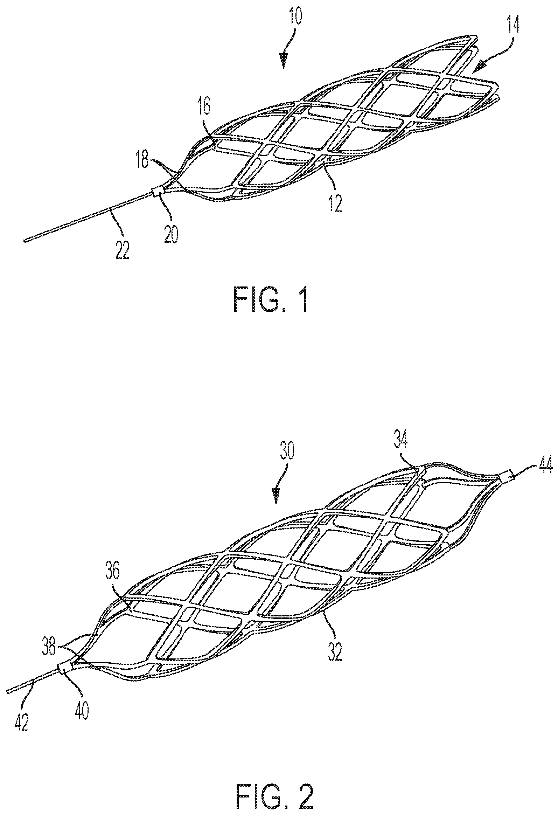

FIGs. 1-26 illustrate multiple embodiments of an embolic capture device of the present teachings. In these figures, the pusher shaft and/or the pull wire are shown along with some exemplary devices for better explanation on their designs and functions, albeit the delivery system that delivers and/or retrieves these exemplary devices are largely not shown.

FIG. 1 shows an embodiment of the embolic capture device (10) of the present teachings in its pre-set deployed configuration. The embolic capture device (10) has a general stent (12) profile with a generally cylindrical body with an open distal end (14). The stent (12) also has a plurality of proximal struts (18), each of which has one end attaching to the proximal end (16) of the cylindrical body, and the other end joining to the other end of another proximal strut (18), forming a proximal hub (20). The proximal hub (20) is configured to allow a positioning wire (not shown) to extend through. According to one embodiment of the present teachings, the cylindrical body of the stent (12) has an open cell structure surface with a central lumen throughout the entire device. The proximal hub (20) of the stent (12) is configured to attach to a distal end of the pusher shaft (22). In one embodiment, the pusher shaft (22) and the positioning wire are configured to extend distally or retract proximally independent of each other.

FIG. 2 illustrates another embodiment of the embolic capture device (30) in its pre-set deployed configuration. Similar to the embodiment shown in FIG. 1, the embolic capture device also has a general stent (32) profile with a general cylindrical body. Unlike the previous embodiments, both the distal (34) and proximal ends (36) of the stent (30) have a plurality of struts (38) which come to a hub (40, 44). According to some embodiments, the proximal hub (40) joins to a distal end of the pusher shaft (42). According to some embodiments, the proximal hub (40) further allows a positioning wire (not shown) to extend there through. According to some embodiments, the distal hub (44) is also configured to allow a positioning wire to extend through (not shown). In one embodiment, the pusher shaft (42) and the positioning wire are configured to extend distally or retract proximally independent of one another.

Further referring to FIGs. 1-2, although not shown, the embolic capture device (10, 30) is configured to be delivered through a microcatheter (not shown). During this delivery process, the distal end of a pusher shaft (22, 42) joins to the proximal hub (20, 40) of the embolic capture device (10, 30). The proximal end of the pusher shaft (20, 40) is controlled by a clinician. By manipulating the pusher shaft (20, 40), the stent (12, 32) can be pushed distally outside of the microcatheter, and retracted proximally back into the larger catheter as needed.

According to one embodiment of the present teachings, the embolic capture device expands upon deployment in vivo. In one embodiment of the present teachings, upon deployment, the embolic capture device expands radially due to the elastic nature of the material. In another embodiment, such radial expansion is achieved by the pre-set thermal shape memory of the device material. In yet another embodiment, such radial expansion is achieved manually via an inflating balloon. In some embodiments, the embolic capture device is made of stainless steel, nitinol, Titanium, Elgiloy, Vitalium, Mobilium, Ticonium, Platinore, Stellite, Tantalum, Platium, Hastelloy, CoCrNi alloys (e.g., trade name Phynox), MP35N, or CoCrMo alloys, any other metallic alloys, or a mixture thereof.

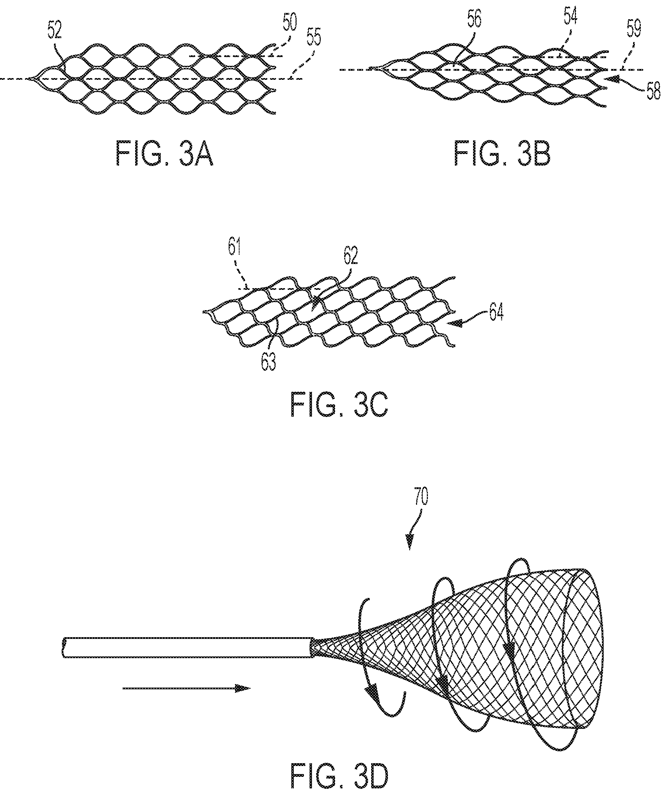

According to some embodiments of the present teachings, the embolic capture device (10, 30) has a stent like shape. In some embodiments, the stent like embolic capture device has a cell structure which allows the stent to collapse during the delivery and expands upon the deployment. FIGs. 3A-3H illustrate several embodiments of the cell structure design on the cylindrical body. FIG. 3A illustrates one embodiment of the cell design. As shown, the cells (52) on the cylindrical body are mostly closed, with the cells (54) at one end of the stent remain open. The cell size are generally uniform throughout the stent with the shape of each cell (52) approximates to a generally square shape. FIG. 3B illustrates another embodiment of the cell design. Similar to what has been described in FIG. 3A, the stent has generally closed cells (56) with uniform size, with open cells (58) at the one end of the stent. Unlike the embodiments of FIG. 3A, the shape of each cell (56) approximates to an elongated diamond shape. FIG. 3C illustrates another embodiment of the cell design. Similar to what has been described in FIG. 3A, the stent has generally closed cells (62) with a uniform size, with open cells (64) at one end of the stent. Unlike the embodiment of FIG. 3A, the shape of each cell (62) approximates to an elongated parallelogram shape.

As illustrated in FIGs. 3A-3B, the cell design of these two exemplary embodiments have the cell axis to be in a horizontal line (as the exemplary dash line 50, 54 shown in FIGs. 3A & 3B), generally parallel to the stent longitudinal axis (55, 59). The cell axis refers to the line formed by connecting the proximal end and distal end of the cell together as shown in FIGs. 3A-3B. In additional, the length of the upper and the lower arcs of each cell are identical for every cell. With this design, the collapsing and expanding of such embolic capture device are generally linear and radially away from the longitudinal axis (55, 59) of the stent.

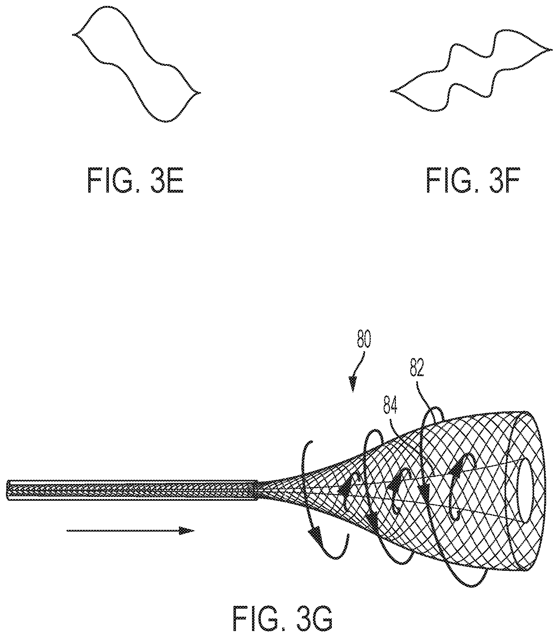

The exemplary embodiment of FIG. 3C illustrates another cell design. Similar to exemplary embodiments shown in FIGs. 3A-3B, the cell axis also are parallel to the stent longitudinal axis. Unlike exemplary embodiments shown in FIGs. 3A-3B, the upper and the lower arcs (61, 63) of each cell (62) are not identical to each other. Each of them (61, 63) follows a different curvature. With this design, the collapsing and expanding of such embolic capture device could be a motion that angles and rotates along the longitudinal axis of the device (70) such as shown in FIG. 3D. In some embodiments, the various height of each cell, length of each cell axis, length of each cell arc, the collapsing and expanding motion of the device can be pre-determined and controlled. For example, the higher the cell height, the greater rotation the device has during collapsing and expanding. In another example, the angle between the cell axis and the longitudinal axis of the device controls the direction of the rotation during collapsing and expanding. For example, when a cell design having a cell axis that is angled to the longitudinal axis of the stent, as shown in FIGs. 3E and 3F. With such angled design, the collapsing and expanding of such embolic capture device could also be a motion that angles and rotates along the longitudinal axis of the device (70) such as shown in FIG. 3D. One skilled in the art should understand that the combination of cell arc length and cell height could control the speed of rotation during collapsing and expanding. One skilled in the art should understand that although FIG. 3C shows that the each cell arc has one smooth curve, other cell arc could also be incorporated such as the ones shown in FIGs. 3E, 3F.

In addition, the embolic capture device could have multiple layers of stent with identical or different cell design. For example as shown in FIG. 3G, the inner layer (84) of the stent (80) could have a cell design allowing the inner layer (84) of the stent (80) to rotate clock wise during expansion and collapsing; and the outer layer (82) of the stent (80) could have a cell design allowing the outer layer (82) of the stent (80) to rotate counter-clock wise during expansion and collapsing. Such multi-layer device design could improve clot engagement and retrieval.

One skilled in the art should understand that the combinations of different cells and/or device design, the device could be programmed to have different movements, such as clock wise rotation, counter-clockwise rotation, zig zag movement, accelerating motion along the length of the device and/or decelerating motion along the length of the device. Thus, the exemplary embodiments as shown in FIGs. 3A-3G, and described herein should not be viewed as limiting to the scope of the present teaching.

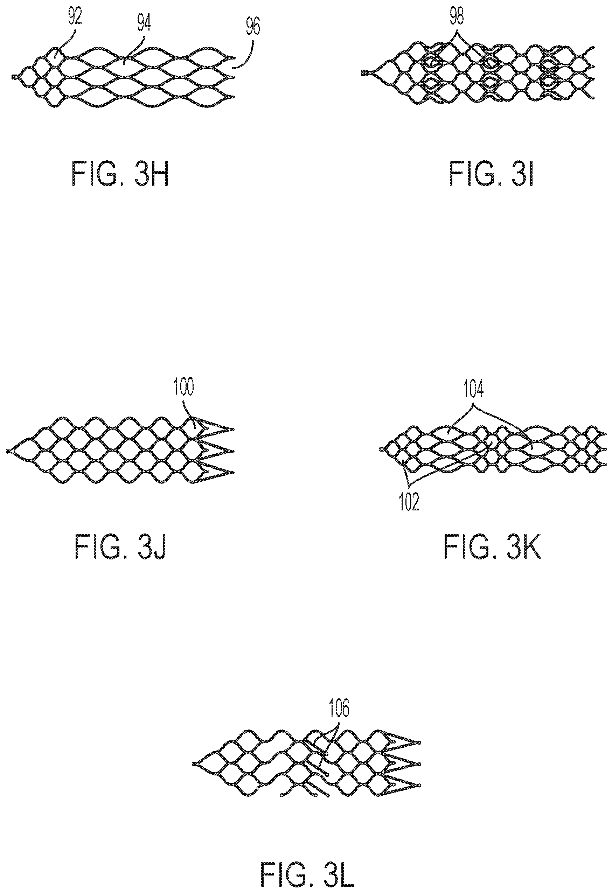

FIG. 3H illustrates another embodiment of the cell design. Similar to the embodiments of FIGs. 3A-3C, the stent has generally closed cells with open cells (96) at the one end of the stent. Unlike the embodiments shown in FIGs. 3A-3C, the stent has small close cells (92) near its closed end, and large close cells (94) throughout middle to the open end sections. According to one embodiment of the present teachings, the larger cell (94) allows the clot to go into the inner lumen of the stent more efficiently, while the smaller cell (92) prevents the smaller emboli from escaping. FIG. 3I illustrates another embodiment of the cell design. Similar to what have been described in FIGs. 3A-3C, the stent has generally closed cells with uniform size, with open cells at the one end of the stent. Similar to what has been described in FIG. 3A, the cell approximates to a generally square shape. In addition, some sections of the stent also have laser cut patterns (98). In some embodiments, the stent has parts (98) that can be heat-set inward. According to one embodiment, these will better capture clots in position. FIG. 3J illustrates another embodiment of the cell design. Unlike the embodiments described in FIGs. 3A-3C, the stent has closed cell throughout its entire section. In some embodiments, the closed end (100) can better capture clots. FIG. 3K illustrates another embodiment of the cell design. Similar to what has been described in FIG. 3H, the stent incorporates larger cell (104) to allow clots to go into the inner lumen of the stent more efficiently, while the smaller cell (102) prevents the smaller emboli from escaping. Unlike the embodiment in FIG. 3H, small cells (102) are incorporated in multiple sections of the stent. FIG. 3L illustrates another embodiment of the cell design. Although this embodiment generally is similar to what has been shown in FIG. 3J, the stent also has c (106) along the stent body, and at the distal end of the stent in order to better capture clots. In one embodiment, fingers (106) along the stent body bend radially inward. And fingers (106) at the distal end of the stent provide more structural integrity to the device.

According to one embodiment of the present teachings, depending on the direction of the axial cell, during the delivery and deployment of an embolic capture device, as the clinician withdraws the microcatheter proximally to uncover the embolic capture device, the device with the cylindrical stent body exits the microcatheter either in a rotating motion or in a straight motion. Similarly, during the emboli retrieval, as the clinician pulls the pusher shaft from its proximal end, the embolic capture device with the cylindrical stent body enters the distal end of the guide catheter either in a rotating motion or in a straight motion. Thus, in some embodiments of the present teachings, it is advantageous for a patient to have an embolic capture device that is designed to intentionally rotate during the delivery, deployment and retrieval. For example, the cylindrical stent body can be designed with a cell structure that allows the embolic capture device to engage to the clots like a screw, as shown in FIG. 3D. In another occasion, it is for the best interest to have an embolic capture device with a minimized profile post deployment movement in order to prevent damage to blood vessel.

One skilled in the art should understand that any combinations of cell size, cell shape, clot capturing fingers, and/or inward parts are all possible. Thus, the specific embodiments described above in reference to FIGs. 3A-3L should not be viewed as limiting to the scope of the present teachings. Additionally, instead of a continuous surface stiffness, in some embodiments, depending on the cell structure, the stiffness of the device could vary from one section to another section. The variable stiffness along the device body will help the stent engage the clot more efficiently.

In some embodiments of the present teachings, the embolic capture device can be fabricated by laser-cutting or acid-etching a pattern into a preformed tube, then shape-setting to the intended deployed configuration. In such embodiments, the embolic capture device can be formed from a hollow tube that has been slotted, for example, by using a machining laser or water drill or other method and then expanded to form an open structure. In another embodiment, the embolic capture device can be formed from wires that are pre-set into the desired shape and then bonded together to connect certain elements on the wires either by cross-hatching, braiding, welding, or other methods of interconnecting rows of metal that are assembled into a tube-like structure. In one embodiment, the wires could be welded using a resistance welding technique or an arc welding technique, preferably while in an inert gas environment and with cooling control to control the grain structure in and around the weld site. These joints can be conditioned after the welding procedure to reduce grain size and their fatigue performance can be optimized by using coining or upset forging.

According to some embodiments of the present teachings, as the embolic capture device being pulled proximally, the device elongates and applies pressure on the clot. In the event where large clots exist, such motion could break the clots, thereby creating multiple emboli. To solve this issue, the stent as shown in FIGs. 1-3 could be modified. One way to solve the problem is to adding a smaller pore size mesh layer to the embolic capture device configured to catch smaller emboli. In one embodiment, the small emboli catching mesh is fixedly attaching to a section of the stent. In another embodiment, the mesh is layered inside or outside of the stent. In yet another embodiment, the mesh is movably incorporating to the stent.

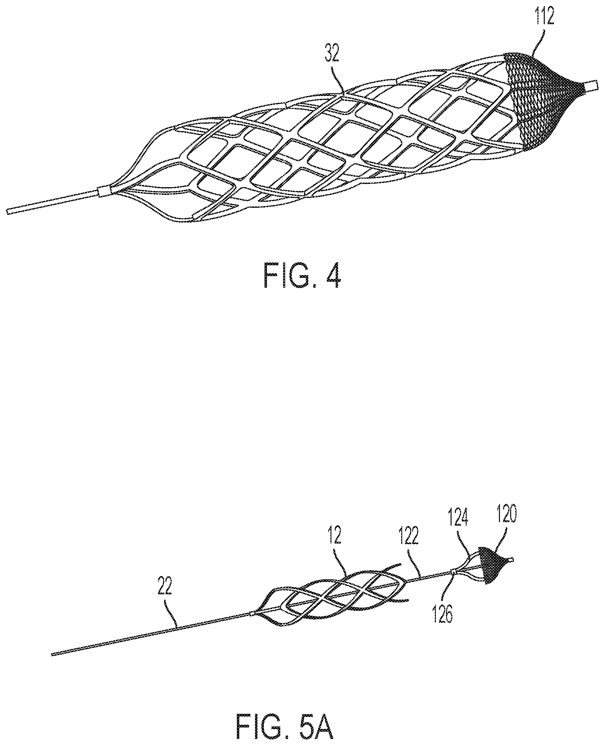

Now referring to FIG. 4, where a mesh net (112) is fixed joining to the embolic capture device (32). According to one embodiment of the present teaching, as shown in the figures, a distal mesh net (112) fixedly attaches to the distal end of the exemplary device as shown in FIG. 2. Such net (112) will help capturing the emboli created during thrombectomy. In one embodiment, the net (112) attaches to the distal end of the device (32) from it outside. In another embodiment, the net (112) attaches to the distal end of the device (32) at it inside. In one embodiment, the mesh net (112) covers the distal struts of the stent. In another embodiment, the mesh net (112) covers the distal struts and a distal portion of the stent.

According to one embodiment of the present teaching, the mesh net can be formed from wire that is pre-bent into the desired shape and then bonded together to connect elements either by welding them or adhesively bonding them. They could be welded using a resistance welding technique or an arc welding technique, preferably while in an inert gas environment and with cooling control to control the grain structure in and around the weld site. These joints can be conditioned after the welding procedure to reduce grain size using coining or upset forging to optimize fatigue performance.

Now referring to FIGs. 5A-5B, where a mesh net (120) is movably joined to the embolic capture device. According to one embodiment of the present teachings, the device has two parts, a stent segment (12) and a distal net segment (120). The embodiments shown in FIGs. 5A-5B are similar to what has been shown in FIG. 1, where the proximal struts join the cylindrical stent body to form a proximal hub. The proximal hub is configured to join a pusher shaft (22). In some embodiments, a pull wire (122) extends through the pusher shaft (22), the proximal hub, and the cylindrical stent body (12). The distal end of the pull wire (122) joins the distal net segment. In some embodiments, the pusher shaft (22) and pull wire (122) move independently from each other.

Continuing to refer to FIGs. 5A-5B, the distal net segment (120) also includes a plurality of struts (124). In some embodiments, the proximal ends of the struts (124) join together to form a hub (126). In some embodiments, the distal ends of the struts (124) join to a distal mesh net (120). In some embodiments, a pull wire (122) joins the distal net (120) at a location. According to some embodiments, in the deployed configuration, the distal net (120) has a deployed profile similar to a cone or in the shape of a tent. In some embodiments, in the deployed configuration, the distal net (120) has a deployed profile similar to an umbrella or an umbrella scaffold. Similar to the stent (12), the proximal hub (126) of the distal net segment (120) is configured to allow a pull wire (122) to extend through. According to one embodiment of the present teachings, the pull wire (122) further extends proximally through the axial lumen of the stent (12), the longitudinal lumen of the pusher shaft (22), and continues outside of the body. In one embodiment, during delivery, the pull wire (122) extends distally, pushes the distal net (120) distally, and collapse the distal net (120) as well as the struts (124) that connect to the net. Thus, the distal net segment (120) assumes an elongated delivery profile where the struts (124) packs closer to one another and are positioned proximally to the distal net (120). In another embodiment, the pull wire (122) retracts proximally, pulls distal net (120) proximally, and thus collapses the distal net (120) as well as the struts (124) that are connected to the net (120). In some embodiments, the distal net segment (120) assumes an elongated delivery profile where the distal net (120) collapses with the struts (124) packed close to one another and are positioned around the collapsed distal net (120). According to one embodiment, during delivery, the stent (12) resumes its elongated delivery profile with the distal net (120) in its elongated delivery profile held inside the axial lumen of the collapsed stent (12). In another embodiment, during delivery, the stent (12) resumes its elongated delivery profile with the distal net (120) in its elongated delivery profile positioned distally to the collapsed stent (12).

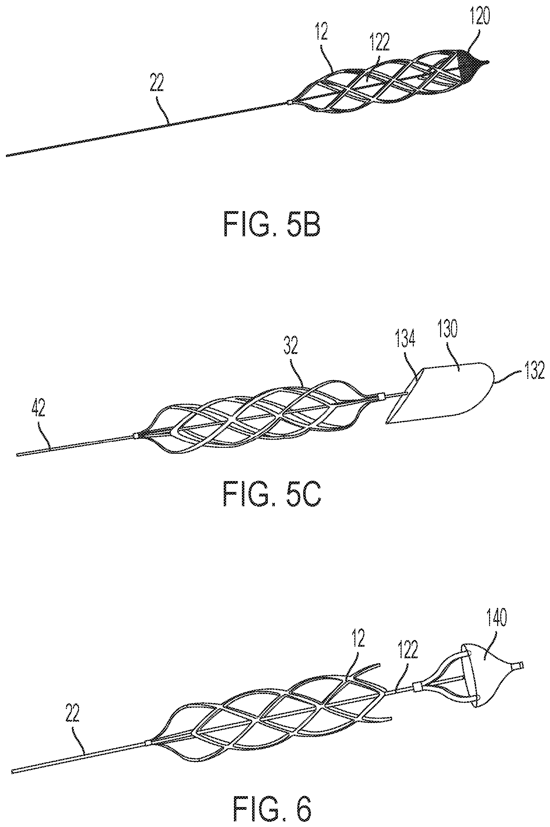

Continuing to refer to FIGs. 5A-5B, once deployed, the distal net (120) is configured to be positioned distally to the stent (12). The distance between the distal net (120) and the stent (12) can be adjusted based on a patient's need. For example, while holding the stent (12) steady, a clinician can push the pull wire (122) distally and thereby move the distal net (120) further away from the stent (12). Alternatively, while holding the stent (12) steady, a clinician can pull the pull wire (122) proximally and move the distal net (120) further away from the stent (12). In one embodiment, as the pusher shaft (22) and pull wire (122) move independently from each other, the distal net (120) has a first configuration where it is axially distal away from the stent (12) as shown in FIG. 5A, the distal net (120) also has the second configuration, where it is partially rest within the distal opening of the stent (12) as shown in FIG. 5B. As shown in FIG. 5B, the struts (124) of the distal net (120) segments are withdrawn inside the distal opening of the stent (12), and at least some portions of the distal net (120) remain outside of the stent (12). During a treatment, a clinician first deploys the stent (12) and then anchors the distal net (120) segment. Once the stent (12) engages the clot, the clinician then moves the distal net (120) closer to the stent (12) to collect the emboli, clot fragments, or leftovers. Such movement can be achieved either by keeping the stent (12) steady, while withdrawing the distal net (120) segment; or by keeping the distal net (120) segment steady while advancing the stent (12).

Although FIGs. 5A-5B illustrate a general cone shaped distal net. One skilled in the art that different profile could also be incorporated to achieve the same functional purpose. For example, as shown in FIG. 5C, the distal net could be in a shape of a mesh tube (130) with a closed distal end (132) and a slanted proximal end (134) that is angled to the longitudinal axis of the mesh tube (130). Thus, one skilled in the art should understand the specific exemplary embodiment shown in these figures and described herein should not be viewed as limiting to the scope of the present teaching. According to one embodiment of the present teaching, the distal net (130) as shown in exemplary embodiments of FIGs. 4-5, could be made of metal material such as nitinol, cobalt, chromium, or other suitable superelastic material

FIG. 6 illustrates a variation to the exemplary embodiments shown in FIGs. 5A-5B. With all other elements similar to what have been described in reference to FIGs. 5A-5B, in this exemplary embodiment, instead of a distal net, the struts of the distal net segment joins a film (140) at their distal end. According to one embodiment, the film (140) could block the blood flow. This configuration allow the device to retain the emboli from flowing distally. Comparing to mesh net, the film (140) could be made of less material with smaller pore. In one embodiment, the film (140) could be made of polymer material such as polyolefin family, nylon, Pebax etc.

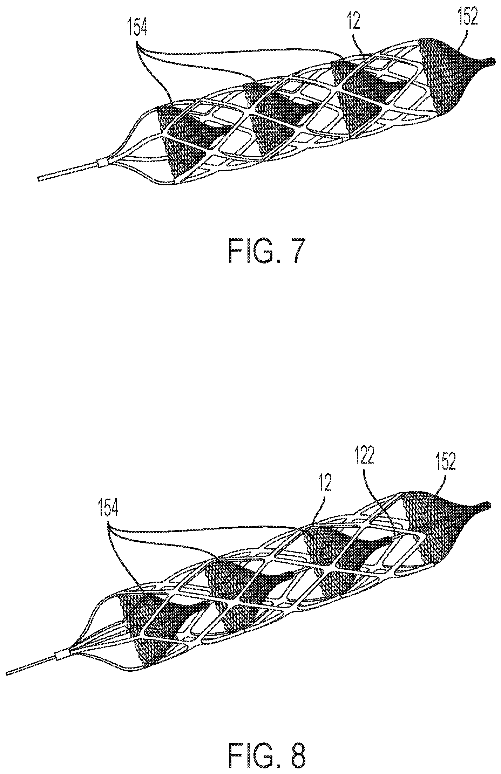

FIGs. 4-6 illustrate exemplary embodiments where only one distal net is incorporated to the stent structure. One skilled in the art should understand that in some embodiments, more than one mesh net could be incorporated. For example, FIG. 7 illustrates the exemplary stent (12) shown in FIG. 1, which not only has a distal net (152) but also incorporates three additional mesh nets (154). As shown in FIG. 7, each net (152, 154) has its bottom edges connecting to the stent (12) and a center portion of the net extends distally away from its bottom edge, so that the mesh net (152, 154) forming a general cone shape. According to one embedment of the present teachings, the mesh net (152, 154) shown in this exemplary embodiment has a pre-formed cone shape, which is configure to collapse for a percutaneous delivery, and assume its expanded profile upon deployment. As shown in FIG.7, each mesh net (152, 154) is contained inside the axial lumen of the stent.

According to another embodiment of the present teachings, a pull wire (122) is configured to extend through and connect to the distal peak of each net for example as shown in FIG. 8. As shown in FIG. 8, the pull wire (122) extends along the longitudinal axis of the stent (12) with the distal end of the pull wire (122) joining the distal peak of the distal net (152, 154). Each mesh net contained inside the stent (12) also joins a section of the pull wire (122). During clot retrieval, all mesh nets (152, 154) act as a cap to catch the debris of the emboli. As a clinician pulls on the proximal end of the pull wire (122), the proximal pulling force transfers to the mesh net (152, 154), making the stent to contain/capture clots more efficiently and minimize the possibility of clot escape.

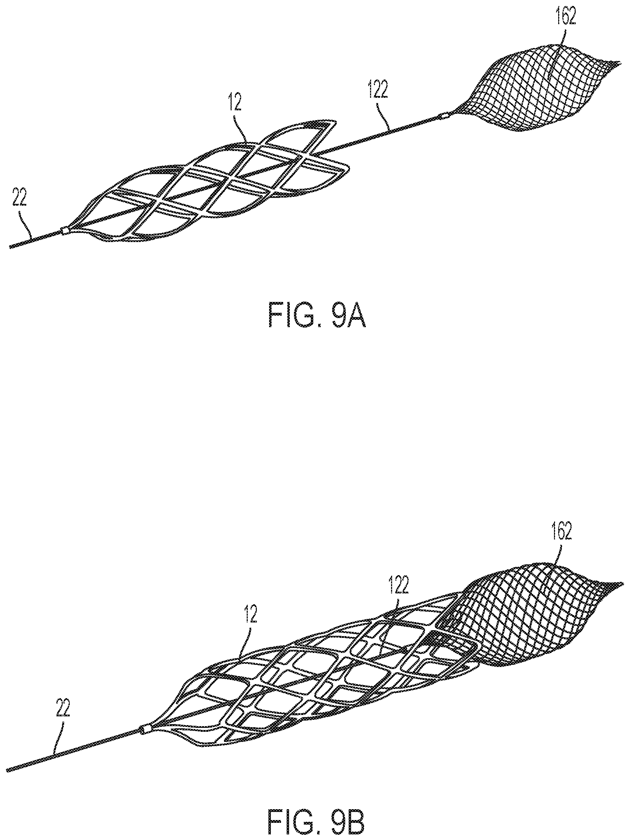

FIGs. 9A-9B illustrate another embodiment described in reference with FIGs. 5A-5B. With all other elements similar to what have been described in reference to FIGs. 5A-5B, in this embodiment, instead of a cone or umbrella scaffold shaped distal net, the distal net (162) has an enclosed 3D profile. In one embodiment, the distal net (162) has a generally round or slightly elongated ball shape, with the pull wire (122) joining to a proximal location of the ball. In another embodiment, such as shown in FIGs. 9A-9B, the distal net (162) is in the shape of a lemon ball. During delivery, the enclosed 3D profile (162) distal net collapses into an elongated configuration which is either placed distally to the collapsed stent (12), or within the axial lumen of the collapsed stent (12). Upon deployment, the enclosed 3D profile distal net (162) expands into a pre-set emboli catching shape.

FIGs. 9A-9B show two deployed configurations of the embolic capture device, which includes the stent (12) and the distal net (162). Similar to what have been described with reference to FIGs. 5A-5B, in one embodiment, as the pusher shaft (22) and the pull wire (122) move independent of each other, the distal net segment (162) has a first configuration where it is axially distally away from the stent (12) as shown in FIG. 9A, the distal net segment (162) also has a second configuration, where it is partially rest within the distal opening of the stent (12) as shown in FIG. 9B. As shown in FIG. 9B, at least a proximal portion of the distal net segment (162) are withdrawn inside the distal opening of the stent (12) with at least a distal portion of the distal net (162) remaining outside of the stent (12). According to one embodiment of the present teachings, the distal net segment (162) is configured to act as a distal protection, a flow restrictor, a laser stent plug, and finally a cleaner when retrieved.

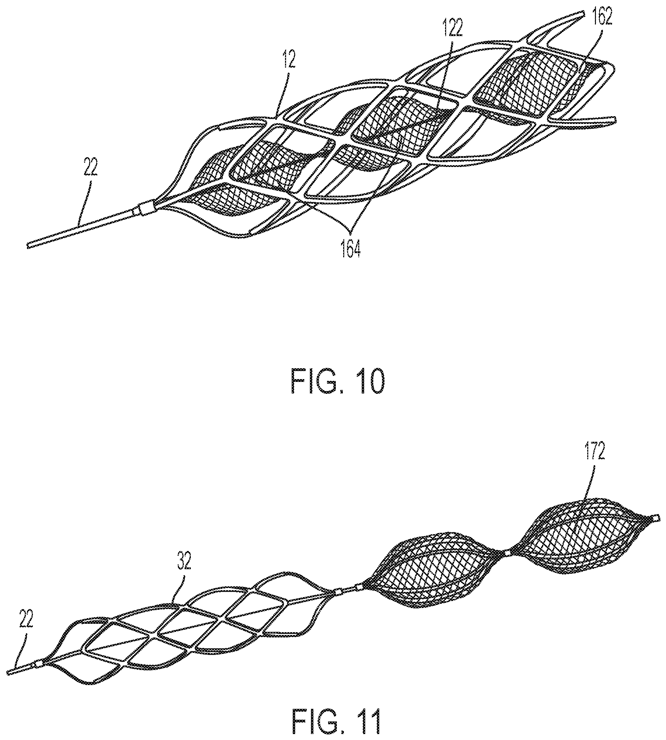

FIG. 10 illustrates a variation of the embodiment shown in FIGs. 9A-9B. With all other elements similar to what have been described in reference to FIGs. 9A-9B, in this embodiment, the distal net segment (162) in this embodiment is made of multiple enclosed 3D mesh nets (162) joining to one another. One skilled in the art should understand that although three enclosed mesh nets (164) are shown in FIG. 10, two, four or even more enclosed mesh nets (164) could be incorporated, thus the scope of present teachings should not be limited to what are illustrated in the drawing. In one embodiment, as illustrated in FIG. 10, the pull wire (122) extends through the proximal enclosed mesh net (164) and joins the proximal end of the very distal enclosed net (162), with two proximal enclosed mesh nets (164) joined each other. In an alternative embodiment, the pull wire (122) joined to the proximal end of the very proximal enclosed mesh net (162) with the all enclosed mesh nets (164) joining to one another.

As the two adjacent enclosed mesh nets (164) join each other, according to one embodiment of the present teaching, the distal end of the proximal enclosed mesh net (164) joins the proximal end of the distal enclosed mesh net (162). With the joining section between two enclosed mesh nets (164) are configured to allow a pull wire (122) to extend through. In one embodiment, each enclosed mesh net (162, 164) deploys independently of each other. Alternatively, all enclosed mesh nets (162, 164) deploy at the same time. In one embodiment, the shape and size of the enclosed mesh nets (162, 164) could be the same. Alternatively, the shape and size of enclosed mesh nets (162, 164) could be different from one another.

Unlike what have been described with reference to FIGs. 9A-9B, the sizes of the enclosed mesh nets (162, 164) are configured such that at a second configuration, all enclosed mesh nets, once deployed, can slide back inside the axial lumen of the stent. The enclosed mesh nets (162, 164) help secure the clots and prevent them from breaking apart once they are captured by the stent.

FIG. 11 illustrates an embodiment of the embolic capture device where an enclosed mesh net (172) is movably joined to the embolic capture device (32) as shown in FIG. 2. Similar to what has been described in reference to FIG. 2, the proximal struts join the cylindrical stent body to form a proximal hub and distal struts join the cylindrical stent body to form a distal hub. As described above, the proximal hub is configured to join a pusher shaft (22), and the distal hub is configured to join a distal net segment (172). Similar to what have been described in reference to FIGs. 9-10, the distal net segment (172) includes at least one enclosed 3D mesh net. According to one embodiment of the present teachings, more than one enclosed mesh nets (172) make up the distal net segment. The configuration of the plurality of enclosed mesh nets are similar to what have been described above, such as with reference to FIGs. 9-10. Similar to what has been described with reference to FIGs. 9A-9B, the distal net segment (172) has a first configuration where it collapses radially, and a second configuration where it expands radially as shown in FIG. 11. According to one embodiment of the present teachings, the proximal stent body (32) is configured to engage, and in some occasion, break up a blood clot; while the distal net segment (172) is configured to clean and catch fragment generated during the procedure. In one embodiment, the stent portion (32) of the device is relatively stiffer than the distal net segment (172) of the device. Although the distal net segment (172) shown in FIGs. 9-11 are described to have a mesh ball, one skilled in the art should understand that depending on the needs, different configurations can be used to increase the effectiveness. For example, the distal net segment could be made of braided mesh, or film.

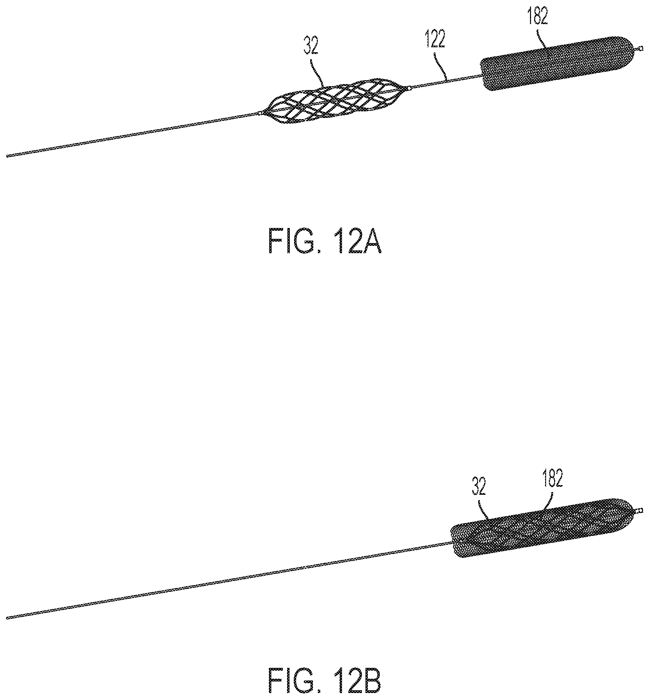

FIGs. 12A-12B illustrate another embodiment of the embolic capture device where a distal net segment is in the profile of a mesh tube (182). The embodiment shown in FIGs. 12A-12B also has a stent (32) similar to what has been described with reference to FIG. 2. Unlike what have been described with reference to FIGs. 9-11, the mesh tube (182) in this embodiment is in the general shape of a test tube with a proximal opening and a distal closed end. The distal end of the pull wire (122) attaches to the distal close end mesh tube (182). Similar to what have been described with reference to FIGs. 9-11, in one embodiment, as the pusher shaft and the pull wire (122) moves independent from each other, the distal net segment (182) has a first configuration where it is axially distally away from the stent (32) as shown in FIG. 12A, the distal net segment (182) also has a second configuration, where it slides over the stent (32) with the mesh tube (182) cover over the at least a portion of the stent (32) as shown in FIG. 12B. As shown in FIG. 12B, in the second configuration, the outer mesh tube (182) covers the inner stent layer. In one embodiment, the mesh tube (182) has the same length as the stent (32). In another embodiment, the mesh tube (182) has a different length than the stent (32). In one embodiment, the mesh tube (182) has an internal diameter relatively the same as the outer diameter of the stent (32). In one embodiment, the mesh tube (182) has a constant diameter throughout its entire length. In another embodiment, the mesh tube (182) varies the diameter from one section to another section. In one embodiment, the proximal end of the mesh tube (182) has a straight profile, generally perpendicular to the longitudinal axis. During emboli retrieval, upon stent body engaging the clot, a clinician then pushes the stent body distally toward the mesh tube (182), thereby preventing all blood emboli fragment from escaping to the blood stream.

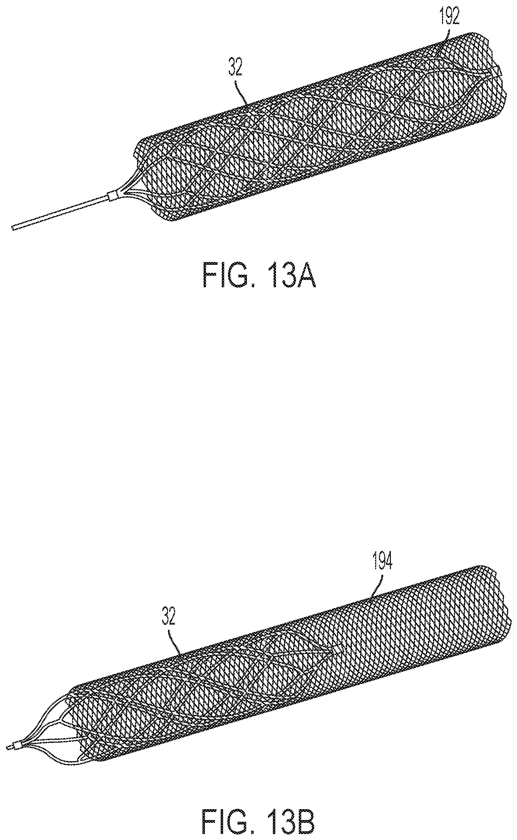

FIGs. 13A-13B illustrate another embodiment of the present teachings. Unlike the embodiments shown in FIG. 12A-12C, the stent (32) is configured to fix to a mesh sleeve layer outside the stent (32) body. In one embodiment, the mesh sleeve (192) has the same length as the stent (32) as shown in FIG. 13A. In another embodiment, the mesh sleeve (192) is longer than the stent (32) as shown in FIG. 13B. According to one embodiment of the present teachings, once the mesh sleeve (192) covers the outside of the stent (32) body, the device will have an increased surface area and improved radial outward force. In another embodiment, where the mesh sleeve (194) is longer than the stent (32) body, such as shown in FIG. 13B, a proximal portion of the stent sleeve (194) covers outside of the stent (32) body, while a distal portion of the mesh sleeve (194) extends over the distal end of the stent (32) body. According to one embodiment of the present teachings, the distal portion of the mesh sleeve (192, 194) is configured to clean and catch emboli fragment generated during the procedure. Similar to embodiments described above, the stent (32) portion of the device is relatively stiffer than the outer mesh sleeve (192, 194) of the device.

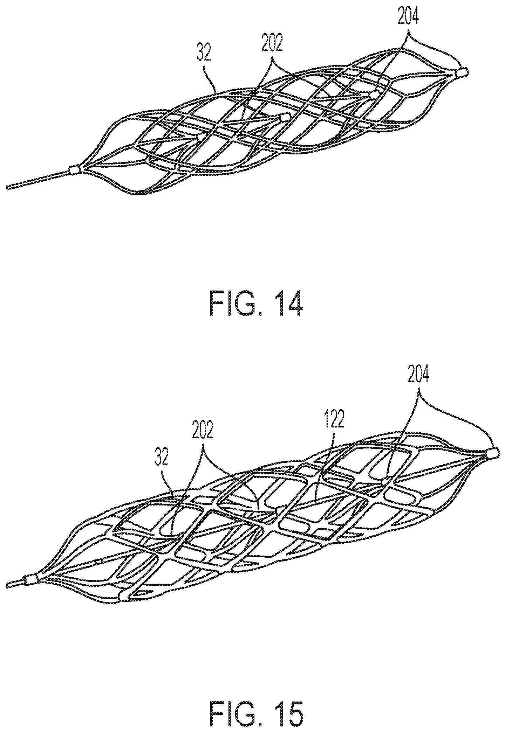

Now, referring to FIGs. 14-15, a stent stabilizing mechanism is employed to the embolic capture device in order to prevent small emboli from escaping from the stent and causing complication. According to some embodiments of the present teachings, during a clot retrieval, once the clot is captured, a retrieval force is applied to the proximal end of the stent, the stent is elongated and its overall diameter shrinks. Thus, to stabilize the stent and prevent the stent wall apposition force from weakening, according to some embodiments of the present teachings, a stabilization strut can be incorporated to keep and even gain more wall apposition during the stent retrieval. According to some embodiments of the present teachings, the stabilizing struts could be used to support the stent structure. As shown in FIG. 14, a stabilizing strut (202) extends from the circumferential wall of the stent (32), extends distally and radially inward at an angle. The distal end (204) of the stabilizing strut (202) ends at a location near the axial center of the stent (32). As shown in the FIG. 14, the distal ends of the first stabilizing strut (202) meet the distal end of another stabilizing strut (202) extending from the same section of the circumferential wall of the stent (32) in a similar manner. Both the ends come together at a location near the axial center of the stent (32). In one embodiment, two stabilizing struts (202) come together to form a set. In another embodiment, more than two stabilizing struts (202) come together to form a set. In one embodiment, two sets of stabilizing struts (202) are incorporated to the stent (32), one set of struts (202) extend from the very distal end of the stent (32), the other set of struts (202) extend from the very proximal end of the stent (32). In another embodiment, more than two sets of stabilizing struts (202) are incorporated with the first set of struts (202) extending from the very distal end of the stent (32), and the second set of struts (202) extending from the very proximal end of the stent (32), and the third set of struts (202) extending from a section of the stent (32) between the distal and proximal ends. One skilled in the art should understand that more than three sets of the stabilizing struts (202) could be incorporated such as the exemplary embodiment shown in FIG. 14, where four sets of stabilizing struts (202) are used.

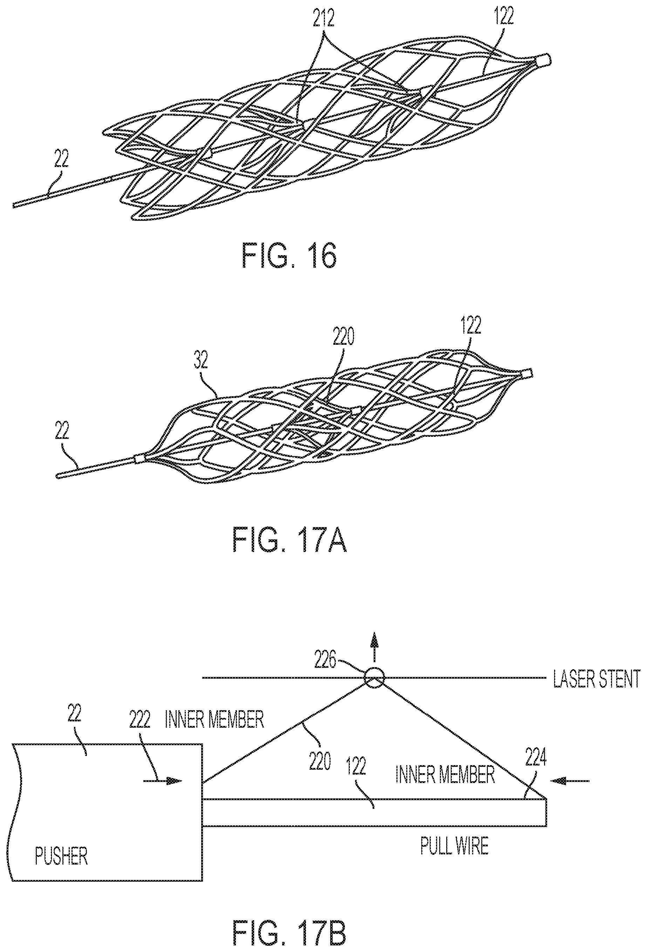

According to some embodiments of the present teachings, at the end of the stabilizing struts (202) where they come together, they also join to the shaft of the pull wire (122) as shown in FIG. 15. In one embodiment, the distal end of each set of the stabilizing struts (202) joins to a section of the shaft of the pull wire (122), as shown in FIG. 15. During the retrieval, while a proximally directional pulling force is applied to the pull wire (122), the stabilizing struts (202) transfer the pull force radially, which force the stent wall to open so that the stent makes better contacts with the clots. FIG. 16 illustrates an alternative embodiment to FIG. 15, where the proximal struts of the stent are removed to increase the efficiency of mechanical thrombectomy process.

FIGs. 17A-17B illustrate another embodiment where stabilizing struts are incorporated to the embolic capture device. According to some embodiments of the present teachings, the stent (32) incorporates at least one directional strut (220). The proximal ends (222) of the struts (220) connect to the distal end of the pusher shaft (22), and the distal ends (224) of the strut (220) connect to a location on the pull wire (122). The middle section of the strut (220) has a joint (226). Such joint (226) attaches to the inner circumferential wall of the stent (32). As shown in FIG. 17A, the pull wire (122) extends through the longitudinal lumen of the pusher shaft (22). The pusher shaft (22) extends distally through the proximal hub of the stent (32) with the distal end of the pusher shaft (22) residing inside the axial lumen of the stent (32). The pusher shaft (22) and pull wire (122) slide independently from each other. According to one embodiment of the present teachings, as the distal end of the pusher shaft (22) moves proximally away from the distal end of the pull wire (122), the two ends of the struts (222, 224) are forced away from each other. As the struts (220) straighten at the joint, the stent (32) wall is pulled radially toward each other. According to another embodiment of the present teachings, as the distal end of the pusher shaft (22) moves distally toward the distal end of the pull wire (122), the two ends of the struts (222, 224) are forced to come close to each other. As the struts (220) bend at their joint, the struts (220) force the stent (32) wall radially outward. This mechanism allows a clinician to adjust the overall size of the stent (32) by manipulating the relative position of the pusher shaft (22) and/or the pull wire (122). According to some embodiments of the present teachings, this design enables a clinician to have control over the overall diameter of the stent by adding and reducing the wall apposition if needed during a clot retrieval. Additionally, this will give the clinician more capability to maneuver through the various diameter blood vessels, and capability to react depending on the clot size.

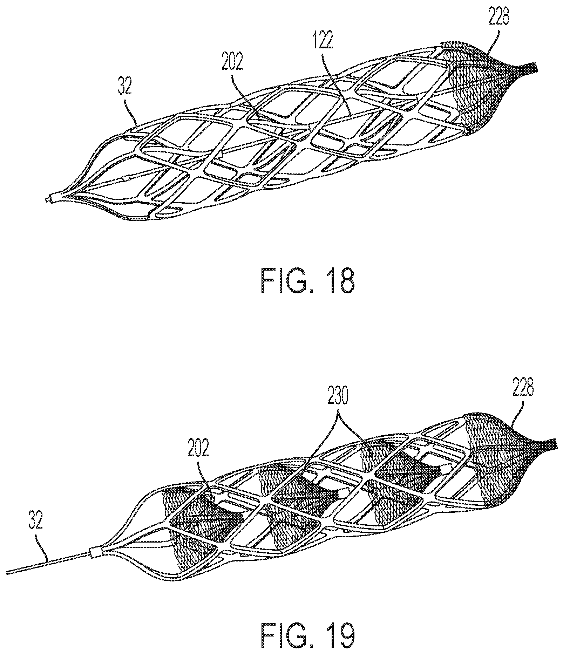

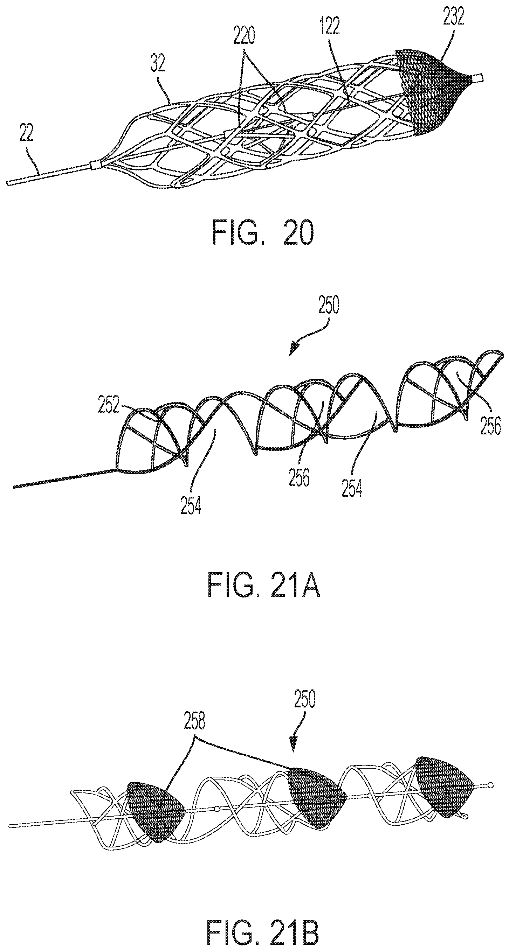

In additional embodiments of the present teachings, similar to what have been described with reference to FIGs. 4-8, a mesh net (228) could be incorporated to the embodiments shown and described with reference to FIGs. 14-17. For example, FIG. 18 illustrates a distal net incorporated to the embodiment shown and described with reference to FIG. 15. FIG. 19 illustrates multiple mesh nets (230) incorporated over a plurality of stabilizing struts (202) sets along with a distal net incorporated to the embodiments shown and described with reference to FIG. 15. FIG. 20 illustrates a distal net (232) incorporated to the embodiments shown and described with reference to FIG. 17A.

FIG. 21A illustrates another embodiment of the embolic capture device (250) according to the present teachings. As illustrated in FIG. 21A, the exemplary stent (252) has a discontinuous pattern (254) throughout the stent surface. As shown, the stent (252) has a continuous portion (256) and a discontinuous portion. The continuous portion (256) has a stent surface character, similar to what has been described above. The discontinuous portion (254) is designed for the clot to travel into the inner lumen of the stent (252). In one embodiment, the discontinuous portion (254) is spirally spaced along the stent luminal surface. For example, the spiral opening is at least one revolution along the stent surface. In another embodiment, the discontinuous portion (254) is linearly spaced along the stent luminal surface. In one embodiment, the discontinuous portion is made of one continuous opening. In another embodiment, the discontinuous portion is made of two or more spiral opening that are connected to each other. In one embodiment, the discontinuous portion takes up more than 50% of the luminal surface. In another embodiment, the discontinuous portion takes up less than 50% of the luminal surface. In one embodiment, the discontinuous portion is arranged from the proximal end of the stent to the distal end of the stent. In another embodiment, the discontinuous portion only locates around one of the distal, proximal and/or middle sections of the stent. In one embodiment, the discontinuous portion has an opening sized similar to the clot to be captured. This design could provide more effective emboli engagement. For example, as the stent being retracted proximally, the clot is enter the inner lumen of the stent once it is contacts the discontinuous portion of the stent. In addition, similar to what has been described above, as shown in FIG. 21B, a mesh net can be further incorporated to the continuous portion of the stent in order for better clot capture.

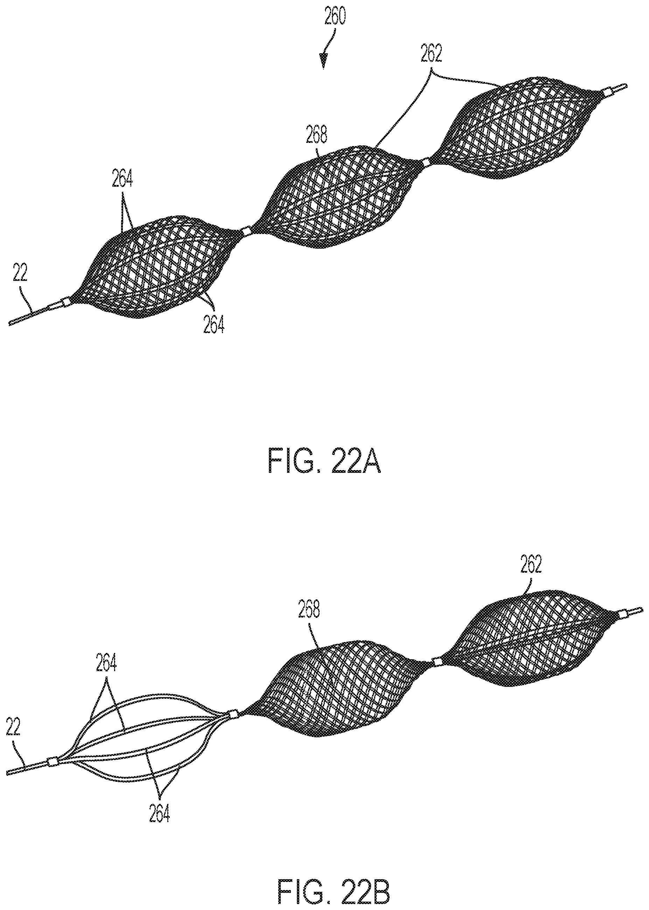

FIG. 22A illustrates another embodiment of the embolic capture device (260) according to the present teachings. As illustrated in FIG. 22A, the exemplary device (260) has a plurality of ball shaped emboli capturing segments (262), one connecting to another in a linear fashion. According to one embodiment, as shown in FIG. 22A, each ball shaped capturing segment (262) has 4 struts (264) in an arc profile extending from the proximal end of the ball segment (262) to the distal end of the ball segment (262). Additionally, the ball shaped segment (262) is covered with mesh net (268). The proximal end of the proximal ball segment (262) is configured to join to a pusher shaft. One skilled in the art should understand that although four struts (264) are illustrated in FIG. 22A, less than four, or more than four struts can be used to build each ball shaped segment. In some embodiments, the ball shaped segment could be made entirely of a mesh net, i.e. with no struts. Additionally, although three ball shaped segments joining together is shown herein, one skilled in the art should understand that more or less than three ball shaped segments could be incorporated to make the complete clot capturing device. Additionally, although all three ball shaped segments (262) are shown to be covered with a mesh net (268), one skilled in the art should understand that one or more ball shaped segments could have their struts exposed without the mesh cover, such as the one shown in FIG. 22B. In one embodiment, all ball shaped segments in the device could have the same configuration, such as the shape and size of each ball, with or without struts, the number of struts incorporated in each ball segment, with or without a mesh cover, the density of the mesh cover, the character of the material used, the type of material used. One skilled in the art should understand that all ball shaped segments in the device could have different configurations.

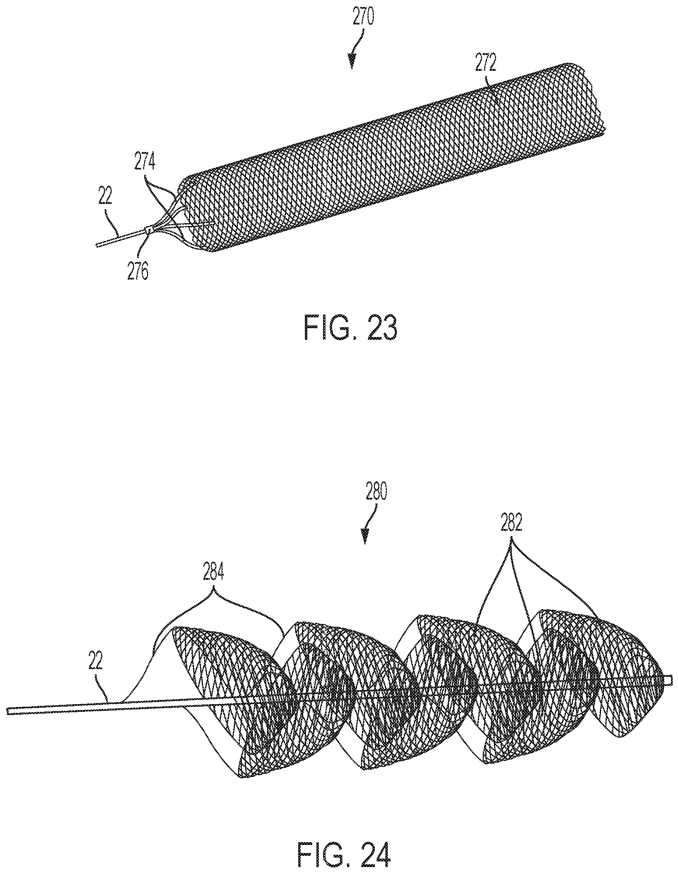

FIG. 23 illustrates another embodiment of the embolic capture device (270) according to the present teachings. As illustrated in FIG. 23, the device has a braided emboli capturing portion with proximal struts (274). All the proximal struts (274) come together to form a proximal hub (276). The distal end of the pusher shaft (22) joins the proximal hub (276) of the device (270). According to one embodiment, the device body can be similar to the mesh sleeve described above, for example those described with reference to FIGs. 13A-13B. During a clot retrieval, upon the clinician's pulling of the pusher shaft (22) proximally, the braided portion elongates and captures the clot.

FIG. 24 illustrates another embodiment of the embolic capture device (280) where the device is mostly made of a mesh net according to the present teachings. As illustrated in FIG. 24, the device has a plurality of mesh nets (282) in the general shape of a basket. Each of the baskets (282) joins the pusher shaft (22) via a strut (284). Said strut (284) joins the opening edge of the strut (284) at its distal end, and a location of the pusher shaft (22) at its proximal end. As shown in FIG. 24, the strut (284) connecting the first basket extends radially away from one side of the pusher shaft (22), with the strut (282) connecting the adjacent basket (282), the second basket (282, distal to the first basket (282) extends radially from the opposite side of the pusher shaft (22). The first basket (282) is proximal to the second basket (282). The first basket (282) partially covers the opening of the second basket (282), as shown in FIG. 24. For example, at least a portion of the first basket (282) extends beyond the opening surface of the second basket (282), while the two adjacent baskets (282) have no direct contact with one another. According to one embodiment of present teachings, this design repeats as many time as desired/designed. One skilled in the art should understand that although FIG. 24 shows seven mesh baskets constituting the entire clot capturing device, more than seven or less than seven baskets could be used to complete a clot capturing device. Thus, the exemplary embodiment shown here should not be viewed as limiting to the scope of the present teachings. In addition, according to one embodiment of the present teachings, each basket is configured to allow the pusher shaft to extend through. The distal end of the pusher shaft (22) joins the distal end of the last distal basket. By extending the pusher shaft distally or retracting the pusher shaft proximally, the device is delivered, deployed, and retrieved once the clot is engaged and captured.

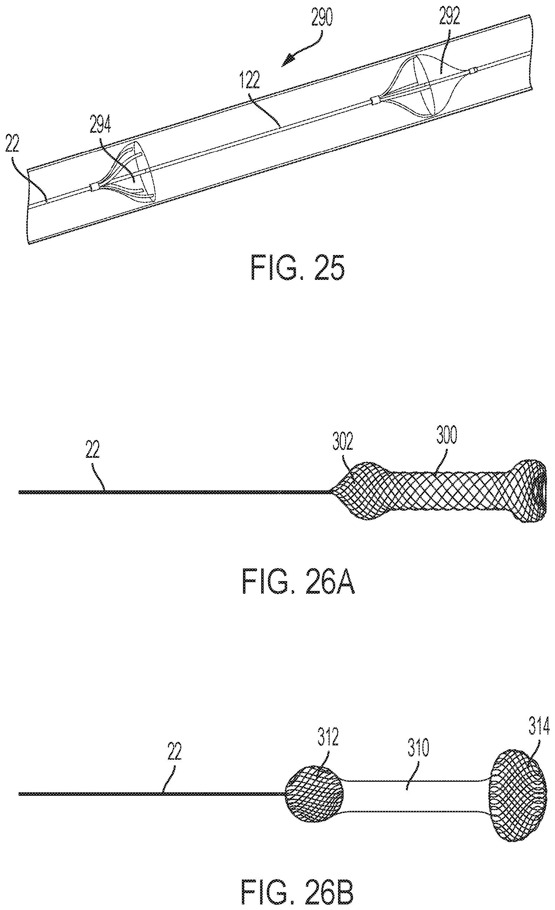

FIG. 25 illustrates another embodiment of the embolic capture device (290) according to the present teachings. As illustrated in FIG. 25, the device has two components, a distal cap (292), and a proximal cap (294). In one embodiment, the distance between the proximal and distal caps (292, 294) is pre-set with the distal cap (292) joining the distal end of the pusher shaft (22), and the proximal cap (294) joining at a location of the pusher shaft (22) from a distance proximally to the distal cap (292). According to another embodiment of the present teachings, the distal end of the pusher shaft (22) joins a proximal cap (294), the distal end of the pull wire (122) joins a distal cap (292). Similar to what has been described above, the pull wire (122) extends through the longitudinal lumen of the pusher shaft (22). The pusher shaft (22) and the pull wire (122) slides independently of each other, so that the distance between the proximal and distal caps (292, 294) are adjustable according to the relative distance between the distal ends of the pusher shaft (22) and pull wire (122).

In application, the distal cap (292) is deployed distally to a clot, the proximal cap (294) is deployed proximally to the clot. While retrieving both the distal and proximal caps, (292, 294) the clot is captured. According to one embodiment of the present teachings, each cap (292, 294) is built with supporting struts covered with either mesh or film layer. In another embodiment, each cap (292, 294) is built with mesh layer alone. In one embodiment, at least one of the proximal and distal caps (292, 294) are configured to self-deploy upon exposed at the treatment location. In another embodiment, at least one of the proximal and distal caps (292, 294) are configured to be manually-deployed at the treatment location. For example, the distal cap (292) could be constructed and deployed in a mechanism similar to an umbrella. As the embodiment shown in FIG. 25 the distal cap (292) has a plurality of supporting struts with the proximal end slidably attaching the pull wire (122), and the distal end attaching to the mesh scaffold, and by pushing the proximal end of the supporting struts distally, the mesh scaffold expands radially.

FIGs. 26A-26B illustrate another embodiment. FIG. 26A illustrates that in additional to an emboli capturing stent (300), a flow restriction mechanism (302) is added to the proximal end of the stent (300). According to one embodiment of the present teachings, the flow restriction (302) reduces the chance of blood clot disruption as a result of restored blood flow. FIG 26B illustrates that in additional to an emboli capturing stent (310), a flow restriction mechanism (312) is added to the proximal end of the stent (310), and distal protection mechanism (314) is added to the distal end of the stent (310). According to one embodiment of the present teachings, the distal protection mechanism catches emboli fragment generated during the procedure. Although as shown in FIGs. 26A-26B, both the distal protection mechanism (314) and the proximal flow restriction mechanism (312) joining to the each end of the stent body (310), have a slightly larger diameter than the general diameter of the stent body (310). One skilled in the art should understand that both the distal protection mechanism (314) and the proximal flow restriction mechanism (312) could have the same or slightly smaller size than the stent body (310). Thus, what has been shown in FIG. 26B should not be viewed as limiting to the scope of the present teachings. In addition, although FIGs. 26A-26B illustrate that both the distal protection mechanism (314) and the proximal flow restriction mechanism (312) are in the configuration of a bulb, one skilled in the art should understand that other profile suitable for achieving the same functional purpose could also be incorporated. In one embodiment, both the distal protection mechanism (314) and the proximal flow restriction mechanism (312) could be made of a mesh, a film. And both the distal protection mechanism (314) and the proximal flow restriction mechanism (312) could be made of the same or different materiel than each other and/or the stent body.

According to some embodiments of the present teachings, the mesh and/or film layer, such as in the shape of a net, a basket, a tube, and/or a sleeve, as described above may be made of a biocompatible metal or polymer metal, polymer braids, laser cut features, porous films, fibers, or more. In some embodiments, the device in whole or portion(s) with curved deployment configuration is made of an elastic material, super-elastic material, or shape-memory alloy which allows said portions to distort into a generally straightened profile during the delivery process and resume and maintain its intended profile in vivo once it is deployed from the delivery catheter. In some embodiments, the device is made of stainless steel, nitinol, Titanium , Elgiloy, Vitalium, Mobilium, Ticonium, Platinore, Stellite, Tantalum, Platium, Hastelloy, CoCrNi alloys (e.g., trade name Phynox), MP35N, or CoCrMo alloys or other metallic alloys. Alternatively, in such embodiments, part or all of the device is made of any flexible, biocompatible material including, but not limited to polyester fabrics, Teflon-based materials, such as ePTFE, UHMPE, HDPE, polypropylene, polysulfone, polyurethanes, metallic materials, polyvinyl alcohol (PVA), extracellular matrix (ECM) isolated from a mammalian tissue, or other bioengineered materials, bioabsorbable polymers such as polyactic acid, polyglycolic acid, polycaprolactone, or other natural materials (e.g., collagen), or combinations of these materials that are well known to those skilled in the art.

According to one embodiment of the present teachings, the mesh/film layer as described above does not impede blood flow through the aperture even though the device assumes a curved deployed profile. According to one embodiment of the present teachings, each mesh opening and the pore size of the film could ranges from 40-300µm. According to another embodiment of the present teachings, the opening surface is 50-95% of entire surface of the mesh layer. According to one embodiment of the present teachings, the mesh net is sized to have a generally same diameter as the stent, such as 1-6mm.

In some embodiments of the present teachings, the mesh layer of the device can be formed of a woven, knitted, or braided tubular metallic fabrics made out of metallic strands. The term "strand" used here can be wires, cords, fibers, yarns, filaments, cables, threads, or the like, and these terms may be used interchangeably. According to one embodiment, the wire used to form the device has a general diameter from about 0.02mm to about 1mm.

According to one embodiment of the present teachings, the mesh layer of the device is fabricated and then shaped to its final configuration. In one embodiment, if a sufficiently elastic and resilient material such as nitinol, is used, the structure can be pre-formed into the finished shape and then elastically deformed and stowed during delivery so the shape will be elastically recovered after deployment. In some embodiments, the mesh layer of the device may be manually expanded to the desired configuration, heat set in an oven while constrained to the desired shape to memorize the desired device shape. According to one embodiment of the present teachings, upon deployment, the mesh layer of the device expands due to the elastic nature of the material. According to another embodiment of the present teachings, upon deployment, mesh layer of the device expands due to its pre-set thermal shape memory of the material.

One skilled in the art will recognize that the device described herein may be used in conjunction with infusing medication through the catheter directly into the thrombus site. Those embodiments with a distal net could limit the medicine, such as tPA (tissue plasminogen activator) that works to dissolve the clot, to be local and directly apply onto the blood clot. Specifically, the distal net of the device creates a closed volume which prevents the tPA from circulating through the system. Since this design also has a function of blocking the blood flow, it prevents blood from further bleeding, injection of medicine, and aspirating the blood out of tissues. For example, the pusher shaft could be configured to excrete tPA or any treatment medication. Those embodiments with a distal net, the space between the stent body and net, or two caps, is configured to create a closed contour which prevents the tPA from circulating systemically. In such event, after a certain amount of time, the emboli will be dissolved with the medicine, by aspiration, and/or retrieved by embolic capture device.

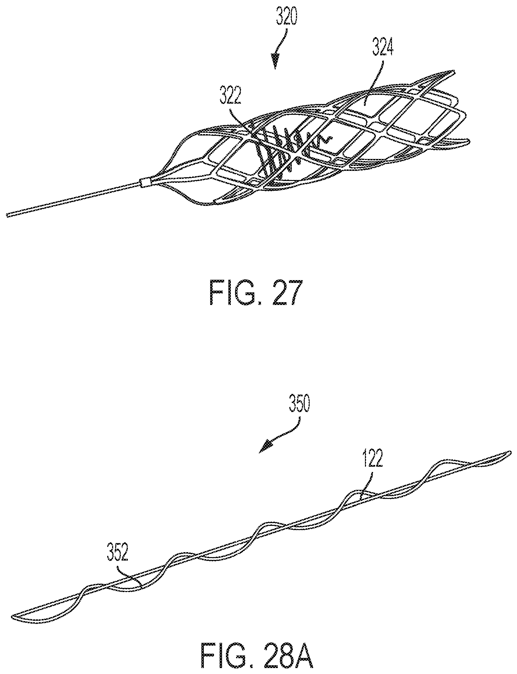

FIG. 27 illustrates another embodiment of the present teachings, where a embolic capture device (320) has a stent body (322) and a vortex coil (324) inside the axial lumen of the stent body (322). As shown in FIG. 27, the vortex coil (324) is made of a continuous wire helically wound into a generally cone shape. The proximal loop of the coil joins the inner luminal wall of the stent body (322). In one embodiment, the proximal loop of the coil joins the proximal section of the stent body (322). In another embodiment, the proximal loop of the coil joins the middle section of the stent body (322). During delivery, the coil (324) is configured to be stretched into an elongated linear shape while remains inside the elongated stent body. Upon deployment, the coil (324) resumes it helically wound cone shape with its distal end remains inside the axial lumen of the stent (322). In one embodiment, the vortex coil (324) allows the clot to be held inside the stent body thereby reducing the possibility of clot breaking off and becoming distal emboli. In one embodiment, the coil could be made of same or different material of the stent body. In another embodiment, the coil could the same or different flexibility as the stent body. Additionally, one skilled in the art should understand that the cone shape as illustrated in FIG. 27 is merely an example, the overall length of the coil, the slope of the cone, and the tightness of each wound, could all vary in order to achieve the intended functionally purpose.

FIGs. 28A-28C illustrate another embodiment of emboli engaging device (350). Such device is used to engage clot for a more efficient retrieval. FIG. 28A illustrates a distal portion of the guide wire (122) with a ribbon (352) wrapped around it in a helical fashion. FIG. 28A illustrates such device in a delivery configuration where the ribbon (352) is tightly wrapped around into an unexpanded state. According to one embodiment of the present teachings, the ribbon (352) is configured to expand radially by an actuation mechanism and engage the clot material. Since the ribbon (352) is wrapped in a helical fashion, as it expands, a portion of the ribbon (352) remains within the clot, a portion of the ribbon (352) extends outside periphery of the clot. This allows the device (350) securely engage the clot. At this point, the device (350) could be retracted proximally carrying the clot outside of the body with or without the assistance of a microcatheter/catheter.

According to one embodiment of the present teachings, the expansion of the ribbon is achieved by a pull wire (not shown) joined to the proximal end of the ribbon (352). As the clinician extends the pull wire distally, the ribbon (352) unwraps from the guidewire, and expands radially. In one embodiment, the pull wire extends along with the guidewire. In another embodiment, the pull wire extends within a longitudinal lumen of the guidewire and comes out at a location and joins the proximal end of the ribbon (352). Such pull wire extends independently to the guidewire. By pulling the pull wire proximally, the device resumes an elongated delivery configuration. By pushing the pull wire distally, the device transitions to an expanded configuration.

In another embodiment of the present teachings, the expansion of the ribbon (352) is achieved by the shape memory character. These ribbon (352) could be heat treated to take a shape. When at the body temperature, these ribbon (352) can resume the pre-set shape. The ribbon (352) can also be configured to be geometrically responsive to an energy input such as electrical current or a wavelength of light. In yet another embodiment, the ribbon could incorporate a combination of two or more of these mechanism.

According to one embodiment, the expansion level of the ribbons could be pre-set or adjustable post implantation. In some embodiments of the present teachings, the cross-section of the ribbon could be circular, rectangular, triangular, elliptical, other shape suitable, or the combination thereof so long it is suitable for the application. In some embodiments, the ribbon could have a hollowed or solid profile. One skilled in the art should understand different ribbon profiles could engender different device-clot interaction properties.

This exemplary embodiment provides an emboli engaging device that could perform thrombectomy without the help of a microcatheter. As such, using the embodiments of the present teachings can reduce vessel traumas, improve effectiveness in tortuosity, increase procedural success rate, and reduce the likelihood of losing the clot in tortuosity.

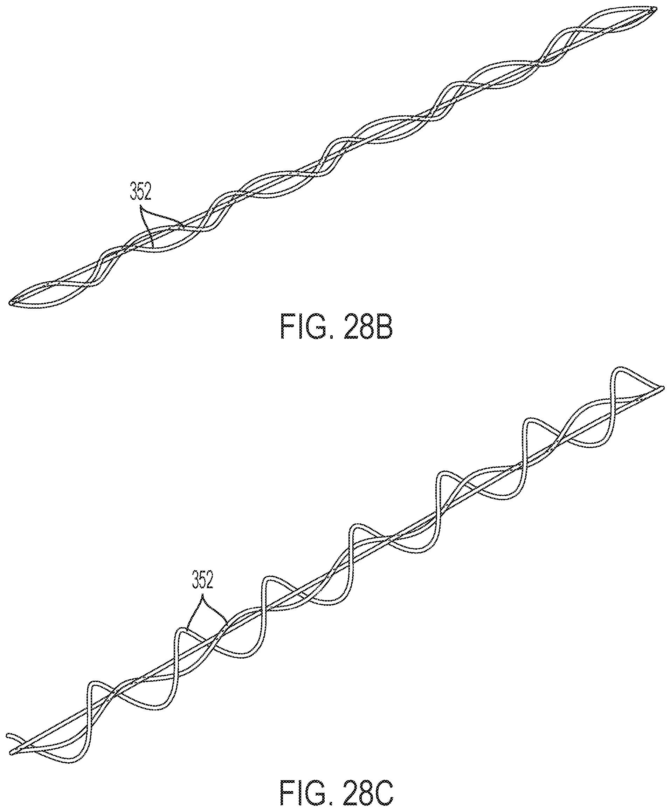

Now referring to FIGs. 28B-28C, a plurality of such ribbons (352) are wrapped along the guide wire. FIG. 28B provides a collapsed delivery profile. FIG. 28C provides an expanded deployed profile. According to one embodiment of the present teachings, all ribbons (352) could have the same shape, size, pitch, and cross-section profile, made of same material, incorporated with the same deployment mechanism, and expands to the same degree. In another embodiment, each ribbon could have a different shape, size, pitch, and cross-section profile, made of different material, incorporated with the different deployment mechanism, and expand to a different degree. According to one embodiment of the present teachings, all ribbons (352) deploys at the same time upon reaching the treatment location. In another embodiment, at least one ribbon deploys at a different pace or time than the rest of the ribbons (352). One skilled in the art should understand that although FIGs. 28B-28C provide two ribbons (352) on a guide wire, more or less than two ribbons (352) could be incorporated so long as it achieves the same desired function.

According to one embodiment of the present teachings, a radioopaque marker is used to make the embolic capture device visible using radiographic imaging equipment such as X-ray, magnetic resonance, ultrasound or other imaging techniques. Markers as disclosed herein may be applied to the ends of any part of the devices, or even on the delivery system of the device. A radioopaque marker can be sewed, adhered, swaged riveted, otherwise placed and secured on the device, The radioopaque marker may be formed of tantalum, tungsten, platinum, irridium, gold, alloys of these materials or other materials that are known to those skilled in the art. The radioopaque marker can also be cobalt, fluorione or numerous other paramagnetic materials or other MR visible materials that are known to those skilled in the arts.

In addition, the delivery system could also be designed for aspiration purpose. For example, the pusher shaft is configured with an aspiration chamber. Such chamber is configured to open for aspiration. Upon delivery the distal end of the pusher shaft to the treatment location, the physician will be able to connect the aspiration pump, or a syringe, to the Pull Hypotube and suck in the clot. In some embodiments, the surface of the pusher shaft with the aspiration chamber is moderated to increase the aspiration effectiveness.

Various embodiments have been illustrated and described herein by way of examples, and one of ordinary skill in the art will appreciate that variations can be made without departing from the scope of the present teachings. The present teachings are capable of other embodiments or of being practiced or carried out in various other ways. Also, it is to be understood that the phraseology and terminology employed herein is for the purpose of description and should not be regarded as limiting.

Unless otherwise defined, all technical and scientific terms used herein have the same meaning as commonly understood by one of ordinary skill in the art to which this present teachings belong. Methods and materials similar or equivalent to those described herein can be used in the practice or testing of the present teachings. In case of conflict, the patent specification, including definitions, will control. In addition, the materials, methods, and examples are illustrative only and not intended to be limiting. The scope of the invention is defined by the claims

## Claims

1. An embolic capture device (30) comprising:
an elongated stent body (32) with a longitudinal axis (55, 59) extending from a proximal end (36) to a distal end (34), a central lumen; a plurality of proximal struts (38) joining the proximal end of elongated stent body forming a proximal hub (40); a plurality of distal struts joining the distal end of elongated stent body forming a distal hub (44); a distal net (120), positioned distally to the elongated stent body; a pusher shaft (42) joining the proximal hub of the elongated stent body; and a pull wire (122) extending through the proximal hub, the elongated stent body, and the distal hub, joining the distal net; wherein the elongated stent body and the distal net are configured to be deployed independently of each other; wherein the elongated stent body comprises a cylindrical body, wherein the cylindrical body comprises a plurality of closed cells (56); **characterised in that** the cell axis is formed by connecting the proximal end and distal end of the cell together is in a horizontal line (50, 54) parallel to the stent longitudinal axis thereby forming upper and the lower arcs (61, 63) of each cell (62), which are not identical and follow a different curvature such that, the collapsing and expanding of such embolic capture device results in a motion that angles and rotates along the longitudinal axis of the device

2. The embolic capture device of claim 1, wherein the elongated stent body comprises a plurality of closed cells along its elongated body and a plurality of open cells at the distal end.

3. The embolic capture device of claim 1, wherein the elongated stent body further comprises a plurality of closed cells along its elongated body, and wherein at least one of the closed cell has a different size than the rest of the closed cell along the elongated stent body.

4. The embolic capture device of claim 2, wherein the elongated stent body further comprises at least one radially inward bending finger toward the central lumen, wherein the at least one radially inward bending finger is configured to capture blood clots.

5. The embolic capture device of claim 1, wherein the pull wire extends through the pusher shaft and is configured to extend and retract independently of the pusher shaft.

6. The embolic capture device of claim 1, wherein the device has a first configuration with a first distance between the distal net and the distal end of the elongated stent body, and a second configuration with a second distance between the distal net and the distal end of the stent body.

7. The embolic capture device of claim 1, wherein the device further comprises a second distal net (112) fixedly attaching to the plurality of distal struts.

8. The embolic capture device of claim 1, wherein the device further comprises at least one mesh net (154) positioned within the central lumen of the elongated stent body.

9. The embolic capture device of claim 8, wherein a proximal edge of the at least one mesh net joins an inner luminal wall of the stent, and a distal peak of the at least one mesh net is located approximate to the longitudinal axis of the elongated stent body.

10. The embolic capture device of claim 9, wherein the pull wire is configured to extend through and connect to the distal peak of the at least one mesh net positioned within the central lumen of the elongated stent body.

11. The embolic capture device of claim 1, wherein the device has a first configuration where the elongated stent body collapses radially, and a second configuration where the elongated stent body expands radially; or , wherein the device has a first configuration where the distal net collapses into an elongated configuration and is positioned distally to the collapsed elongated stent body.

12. The embolic capture device of claim 1, wherein the distal net is in the shape of a mesh tube with a proximal opening and a closed distal end, and wherein the device has a first configuration where the mesh tube is axially and distally away from the elongated stent body, and a second configuration where the mesh tube slides over and covers at least a portion of the elongated stent body; or, wherein the distal net is in the shape of a mesh sleeve with a distal opening and a proximal opening, and wherein the device has a first configuration where the mesh sleeve is axially and distally away from the elongated stent body, and a second configuration where the mesh sleeve slides over and covers at least a portion of the elongated stent body.

13. The embolic capture device of claim 1, wherein the device further comprises at least a pair of stabilizing struts extending from the same section of the circumferential wall of the elongated stent body distally and radially inward at an angle toward the central lumen, wherein both distal ends of the at least a pair of stabilizing struts join together at a location approximate to the elongated axial of the elongated stent body, optionally, wherein the pull wire joins the distal ends of the at least a pair of stabilizing struts, and is configured to apply a proximal pulling force to the at least one pair of stabilizing struts.

14. The embolic capture device of claim 1, wherein the device further comprises at least one directional strut, wherein a proximal end of the directional strut connects to a distal end of the pusher shaft, a distal end of the directional strut connects to a distal end of the pull wire, and a middle joint of the directional strut attaches to an inner circumferential wall of the elongated stent body, and wherein as the device has a first configuration which as the distal ends of the pusher shaft and the pull wire moving away from each other, the at least one directional strut straightens, and the elongated stent body collapses radially; and a second configuration which as the distal ends of the pusher shaft and the pull wire moving toward each other, the at least one directional strut bends, and the elongated stent body expands radially.

## Patentansprüche

1. Embolie-Einfangvorrichtung (30), umfassend:
einen länglichen Stentkörper (32) mit einer Längsachse (55, 59), die sich von einem proximalen Ende (36) zu einem distalen Ende (34) erstreckt, ein zentrales Lumen; eine Vielzahl von proximalen Streben (38), die mit dem proximalen Ende des länglichen Stentkörpers verbunden ist und eine proximale Nabe (40) ausbildet; eine Vielzahl von distalen Streben, die mit dem distalen Ende des länglichen Stentkörpers verbunden ist und eine distale Nabe (44) ausbildet; ein distales Netz (120), das distal zu dem langgestreckten Stentkörper positioniert ist; einen Drückerschaft (42), der mit der proximalen Nabe des länglichen Stentkörpers verbunden ist; und einen Zugdraht (122), der sich durch die proximale Nabe, den länglichen Stentkörper und die distale Nabe erstreckt und mit dem distalen Netz verbunden ist; wobei der längliche Stentkörper und das distale Netz konfiguriert sind, um unabhängig voneinander entfaltet zu werden; wobei der längliche Stentkörper einen zylindrischen Körper umfasst, wobei der zylindrische Körper eine Vielzahl von geschlossenen Zellen (56) umfasst; **dadurch gekennzeichnet, dass** die Zellenachse, die durch Verbinden des proximalen Endes und des distalen Endes der Zelle ausgebildet ist, in einer horizontalen Linie (50, 54) parallel zur Stent-Längsachse liegt, wodurch ein oberer und ein unterer Bogen (61, 63) jeder Zelle (62) ausgebildet werden, die nicht identisch sind und einer unterschiedlichen Krümmung folgen, sodass das Zusammenklappen und Ausdehnen einer solchen Embolie-Einfangvorrichtung zu einer Bewegung führt, die entlang der Längsachse der Vorrichtung einen Winkel bildet und sich dreht.

2. Embolie-Einfangvorrichtung nach Anspruch 1, wobei der längliche Stentkörper eine Vielzahl von geschlossenen Zellen entlang seines länglichen Körpers und eine Vielzahl von offenen Zellen an dem distalen Ende umfasst.

3. Embolie-Einfangvorrichtung nach Anspruch 1, wobei der längliche Stentkörper ferner eine Vielzahl von geschlossenen Zellen entlang seines länglichen Körpers umfasst und wobei mindestens eine der geschlossenen Zellen eine andere Größe als die übrigen geschlossenen Zellen entlang des länglichen Stentkörpers aufweist.

4. Embolie-Einfangvorrichtung nach Anspruch 2, wobei der längliche Stentkörper ferner mindestens einen radial nach innen in Richtung des zentralen Lumens gebogenen Finger umfasst, wobei der mindestens eine radial nach innen gebogene Finger zum Einfangen von Blutgerinnseln konfiguriert ist.

5. Embolie-Einfangvorrichtung nach Anspruch 1, wobei sich der Zugdraht durch den Drückerschaft erstreckt und konfiguriert ist, um sich unabhängig von dem Drückerschaft zu verlängern und zurückzuziehen.

6. Embolie-Einfangvorrichtung nach Anspruch 1, wobei die Vorrichtung eine erste Konfiguration mit einem ersten Abstand zwischen dem distalen Netz und dem distalen Ende des länglichen Stentkörpers und eine zweite Konfiguration mit einem zweiten Abstand zwischen dem distalen Netz und dem distalen Ende des Stentkörpers aufweist.

7. Embolie-Einfangvorrichtung nach Anspruch 1, wobei die Vorrichtung ferner ein zweites distales Netz (112) umfasst, das fest an der Vielzahl von distalen Streben befestigt ist.

8. Embolie-Einfangvorrichtung nach Anspruch 1, wobei die Vorrichtung ferner mindestens ein Maschennetz (154) umfasst, das innerhalb des zentralen Lumens des länglichen Stentkörpers positioniert ist.

9. Embolie-Einfangvorrichtung nach Anspruch 8, wobei eine proximale Kante des mindestens einen Maschennetzes mit einer inneren Lumenwand des Stents verbunden ist und sich ein distaler Scheitelpunkt des mindestens einen Maschennetzes in der Nähe der Längsachse des länglichen Stentkörpers befindet.

10. Embolie-Einfangvorrichtung nach Anspruch 9, wobei der Zugdraht konfiguriert ist, um sich durch den distalen Scheitelpunkt des mindestens einen innerhalb des zentralen Lumen des länglichen Stentkörpers positionierten Maschennetzes zu erstrecken und sich mit diesem zu verbinden.

11. Embolie-Einfangvorrichtung nach Anspruch 1, wobei die Vorrichtung eine erste Konfiguration, in der der längliche Stentkörper radial zusammenklappt, und eine zweite Konfiguration, in der sich der längliche Stentkörper radial ausdehnt, aufweist; oder wobei die Vorrichtung eine erste Konfiguration aufweist, in der das distale Netz zu einer länglichen Konfiguration zusammenklappt und distal zu dem zusammengeklappten länglichen Stentkörper positioniert ist.

12. Embolie-Einfangvorrichtung nach Anspruch 1, wobei das distale Netz die Form eines Maschenschlauchs mit einer proximalen Öffnung und einem geschlossenen distalen Ende aufweist und wobei die Vorrichtung eine erste Konfiguration, in der der Maschenschlauch axial und distal von dem länglichen Stentkörper entfernt ist, und eine zweite Konfiguration, in der der Maschenschlauch über den länglichen Stentkörper gleitet und mindestens einen Abschnitt davon bedeckt, aufweist; oder wobei das distale Netz die Form einer Maschenhülse mit einer distalen Öffnung und einer proximalen Öffnung aufweist und wobei die Vorrichtung eine erste Konfiguration, in der die Maschenhülse axial und distal von dem länglichen Stentkörper entfernt ist, und eine zweite Konfiguration, in der die Maschenhülse über den länglichen Stentkörper gleitet und mindestens einen Abschnitt davon bedeckt, aufweist.

13. Embolie-Einfangvorrichtung nach Anspruch 1, wobei die Vorrichtung ferner mindestens ein Paar von Stabilisierungsstreben umfasst, das sich von demselben Teilabschnitt der Umfangswand des länglichen Stentkörpers distal und radial nach innen in einem Winkel in Richtung des zentralen Lumens erstreckt, wobei die beiden distalen Enden des mindestens einen Paars von Stabilisierungsstreben an einer Stelle in der Nähe der länglichen Axialen des länglichen Stentkörpers verbunden sind, wobei optional der Zugdraht die distalen Enden des mindestens einen Paars von Stabilisierungsstreben verbindet und konfiguriert ist, um eine proximale Zugkraft auf das mindestens eine Paar von Stabilisierungsstreben auszuüben.

14. Embolie-Einfangvorrichtung nach Anspruch 1, wobei die Vorrichtung ferner mindestens eine Richtungsstrebe umfasst, wobei sich ein proximales Ende der Richtungsstrebe mit einem distalen Ende des Drückerschafts verbindet, sich ein distales Ende der Richtungsstrebe mit einem distalen Ende des Zugdrahts verbindet und eine Mittelverbindung der Richtungsstrebe an einer inneren Umfangswand des länglichen Stentkörpers befestigt ist, und wobei, wenn die Vorrichtung eine erste Konfiguration aufweist, in der sich die distalen Enden des Drückerschafts und des Zugdrahts voneinander weg bewegen, die mindestens eine Richtungsstrebe gerade wird und der längliche Stentkörper radial zusammenklappt; und bei einer zweiten Konfiguration, in der sich die distalen Enden des Drückerschafts und des Zugdrahts aufeinander zubewegen, sich die mindestens eine Richtungsstrebe biegt und sich der längliche Stentkörper radial ausdehnt.

## Revendications

1. Dispositif de capture embolique (30) comprenant :
un corps de stent allongé (32) avec un axe longitudinal (55, 59) s'étendant d'une extrémité proximale (36) à une extrémité distale (34), une lumière centrale ; une pluralité d'entretoises proximales (38) rejoignant l'extrémité proximale du corps de stent allongé pour former un moyeu proximal (40) ; une pluralité d'entretoises distales rejoignant l'extrémité distale du corps de stent allongé pour former un moyeu distal (44) ; un filet distal (120), positionné distalement par rapport au corps de stent allongé ; une tige de poussée (42) rejoignant le moyeu proximal du corps de stent allongé ; et un fil de traction (122) s'étendant à travers le moyeu proximal, le corps de stent allongé et le moyeu distal, rejoignant le filet distal ; dans lequel le corps de stent allongé et le filet distal sont configurés pour être déployés indépendamment l'un de l'autre ; dans lequel le corps allongé du stent comprend un corps cylindrique, dans lequel le corps cylindrique comprend une pluralité de cellules fermées (56) ; **caractérisé en ce que** l'axe de cellule est formé en reliant ensemble l'extrémité proximale et l'extrémité distale de la cellule sur une ligne horizontale (50, 54) parallèle à l'axe longitudinal du stent, formant ainsi les arcs supérieur et inférieur (61, 63) de chaque cellule (62), qui ne sont pas identiques et présentent des courbures différentes, de sorte que le déploiement et le repliement d'un tel dispositif de capture embolique induisent un mouvement angulaire et rotatif autour de l'axe longitudinal du dispositif.

2. Dispositif de capture embolique selon la revendication 1, dans lequel le corps allongé du stent comprend une pluralité de cellules fermées le long de son corps allongé et une pluralité de cellules ouvertes au niveau de l'extrémité distale.

3. Dispositif de capture embolique selon la revendication 1, dans lequel le corps allongé du stent comprend en outre une pluralité de cellules fermées le long de son corps allongé, et dans lequel au moins l'une des cellules fermées a une taille différente du reste des cellules fermées le long du corps allongé du stent.

4. Dispositif de capture embolique selon la revendication 2, dans lequel le corps allongé du stent comprend en outre au moins un doigt courbé radialement vers l'intérieur en direction de la lumière centrale, dans lequel l'au moins un doigt courbé radialement vers l'intérieur est configuré pour capturer des caillots sanguins.

5. Dispositif de capture embolique selon la revendication 1, dans lequel le fil de traction s'étend à travers la tige de poussée et est configuré pour s'étendre et se rétracter indépendamment de la tige de poussée.

6. Dispositif de capture embolique selon la revendication 1, dans lequel le dispositif présente une première configuration avec une première distance entre le filet distal et l'extrémité distale du corps allongé du stent, et une seconde configuration avec une seconde distance entre le filet distal et l'extrémité distale du corps du stent.

7. Dispositif de capture embolique selon la revendication 1, dans lequel le dispositif comprend en outre un second filet distal (112) attaché fixement à la pluralité d'entretoises distales.

8. Dispositif de capture embolique selon la revendication 1, dans lequel le dispositif comprend en outre au moins un filet maillé (154) positionné dans la lumière centrale du corps allongé du stent.

9. Dispositif de capture embolique selon la revendication 8, dans lequel un bord proximal de l'au moins un filet maillé rejoint une paroi luminale interne du stent, et un sommet distal de l'au moins un filet maillé est situé approximativement sur l'axe longitudinal du corps allongé du stent.

10. Dispositif de capture embolique de la revendication 9, dans lequel le fil de traction est configuré pour s'étendre à travers et se connecter au sommet distal de l'au moins un filet maillé positionné dans la lumière centrale du corps allongé du stent.

11. Dispositif de capture embolique selon la revendication 1, dans lequel le dispositif présente une première configuration dans laquelle le corps allongé du stent se replie radialement, et une seconde configuration dans laquelle le corps allongé du stent s'étend radialement ; ou, dans lequel le dispositif présente une première configuration dans laquelle le filet distal se replie dans une configuration allongée et est positionné distalement par rapport au corps allongé replié du stent.

12. Dispositif de capture embolique selon la revendication 1, dans lequel le filet distal a la forme d'un tube maillé avec une ouverture proximale et une extrémité distale fermée, et dans lequel le dispositif présente une première configuration dans laquelle le tube maillé est axialement et distalement éloigné du corps allongé du stent, et une seconde configuration dans laquelle le tube maillé glisse sur et recouvre au moins une partie du corps allongé du stent ; ou, dans lequel le filet distal a la forme d'un manchon maillé avec une ouverture distale et une ouverture proximale, et dans lequel le dispositif présente une première configuration dans laquelle le manchon maillé est axialement et distalement éloigné du corps allongé du stent, et une seconde configuration dans laquelle le manchon maillé glisse sur et recouvre au moins une partie du corps allongé du stent.

13. Dispositif de capture embolique selon la revendication 1, dans lequel le dispositif comprend en outre au moins une paire d'entretoises stabilisatrices s'étendant à partir de la même section de la paroi circonférentielle du corps allongé du stent, distalement et radialement vers l'intérieur, selon un angle vers la lumière centrale, dans lequel les deux extrémités distales de l'au moins une paire d'entretoises stabilisatrices se rejoignent à un emplacement approximativement au niveau de l'axe allongé du corps allongé du stent, éventuellement, dans lequel le fil de traction rejoint les extrémités distales de l'au moins une paire d'entretoises stabilisatrices, et est configuré pour appliquer une force de traction proximale à l'au moins une paire d'entretoises stabilisatrices.

14. Dispositif de capture embolique selon la revendication 1, dans lequel le dispositif comprend en outre au moins une entretoise directionnelle, dans lequel une extrémité proximale de l'entretoise directionnelle est reliée à une extrémité distale de la tige de poussée, une extrémité distale de l'entretoise directionnelle est reliée à une extrémité distale du fil de traction, et une articulation centrale de l'entretoise directionnelle est fixée à une paroi circonférentielle interne du corps allongé du stent, et dans lequel, étant donné que le dispositif présente une première configuration dans laquelle, lorsque les extrémités distales de la tige de poussée et du fil de traction s'éloignent l'une de l'autre, l'au moins une entretoise directionnelle se redresse et le corps allongé du stent se rétracte radialement ; et une seconde configuration dans laquelle, lorsque les extrémités distales de la tige de poussée et du fil de traction se rapprochent, l'au moins une entretoise directionnelle se courbe et le corps allongé du stent s'étend radialement.
